# EUROPEAN PATENT SPECIFICATION

(11) **EP 0 820 489 B1**
(45) Date of publication and mention of the grant of the patent: **11.07.2001**
(21) Application number: 96909805.2
(22) Date of filing: 22.03.1996
(51) Int. Cl.: C09B 47/04, G01N 33/533, C07D 487/22, C07F 7/08

(54) **HYBRID PHTHALOCYANINE DERIVATIVES AND THEIR USES**
HYBRIDE PHTALOCYANINEDERIVATEN UND DEREN VERWENDUNGEN
DERIVES HYBRIDES DE PHTHALOCYANINE ET LEURS UTILISATIONS

(30) Priority: 23.03.1995 US 409825
(43) Date of publication of application: 28.01.1998
(73) Proprietor: BIOSITE DIAGNOSTICS INC., San Diego California 92121 (US)
(72) Inventor: BUECHLER, Kenneth, F., San Diego, CA 92071 (US); NOAR, Joseph, B., Solana Beach, CA 92075 (US); TADESSE, Lema, San Diego, CA 92126 (US)
(74) Representative: Viering, Jentschura & Partner
(86) International application number: US9603833
(87) International publication number: WO9629367

(56) References cited:
- EP-A- 0 285 965
- EP-A- 0 597 389
- WO-A-91/18007
- WO-A-93/19366
- WO-A-95/08772

## Description

This invention relates generally to the synthesis of novel dyes and labels and methods for the detection or visualization of analytes and more specifically to the detection of analytes in immunoassays or in nucleic acid assays.

### BACKGROUND ART

Various methodologies are available for the visualization of cells or molecules in cells and for the measurement of analyte concentrations in fluids. Fluorescence microscopy utilizes fluorescent dyes, generally connected to specific probes, such as antibodies, for the localization of proteins and complexes in cells. For the measurement of analyte concentrations, immunoassays have become popular over the last 40 years because of the specificity of antibodies toward the analyte or target ligand. Radioimmunoassays were developed because the high specific activity of the radionucleotide allowed measurement of very low concentrations of analyte. However, because of the concerns for the environment and human health, the use of radionucleotides in immunoassays is becoming less popular. The use of enzymes in immunoassays to amplify a signal has been a very important advance in the field of immunoassays because their use does not involve environmental or human health hazards or risks. Enzyme-linked immunoassays, however, can be problematic because the activity of the enzyme is temperature dependent and the instability of the enzyme or the substrates can result in inaccurate quantitation of the target ligand. Still other immunoassays monitor fluorescence as the signal, with or without enzymes, for the measurement of analyte concentrations.

The characteristics of the fluorescent dyes are very important when quantifying analyte concentrations in biological fluids. For example, when the biological fluid is blood, serum or plasma, the intrinsic fluorescence of the fluid precludes the use of many dyes. These biological fluids generally have fluorescence emissions up to 600 nm when exciting at various wavelengths above 200 nm. The fluorescence is generated by excitation of the dye at the appropriate wavelength. The fluorescent signal is measured by a fluorometer which is tuned to excite the fluorescent molecule at a specific wavelength and to measure the emission of fluorescence at another wavelength. The difference in the excitation and emission wavelengths is referred to as the Stokes shift. To achieve the most sensitive measurement, the emission wavelength of the sample should not interfere with the emission of the dye. Also, the Stokes shift should be as large as possible so that the excitation light is not seen by the detector as a background signal. When the Stokes shift is not large, filters or monochromators can be utilized in the fluorometer to exclude light near the emission wavelength; however, the use of filters decreases the yield of light reaching the detector and generally one circumvents this problem of light loss by the use of high intensity lamps. Thus, to avoid problems associated with small Stokes shifts and dyes which emit near the intrinsic emission of the biological fluid, a sophisticated instrument is generally built. With the advent of near-patient diagnostics in hospitals, there is a need for portable, simple fluorometers which can assess fluorescence in an immunoassay for the detection of analytes in biological samples.

Another problem associated with the assay of analytes in fluids or the visualization of cellular components with an intrinsic fluorescence is that of selection of the dye which is utilized as the label. The dye is generally chosen for its brightness (the product of fluorescence quantum yield and extinction coefficient) since a certain sensitivity in the assay or the visualization technique is required. However, the selection of the dye used as the label is limited when the sample has an intrinsic fluorescence because the instrument may not be capable of distinguishing sample fluorescence from dye fluorescence.

The current invention provides a methodology for the development of amplified fluorescent label systems which can be tuned to specific excitation and emission wavelengths. The methodology teaches the design and synthesis of novel hybrid phthalocyanine derivatives which are synthesized as water-soluble molecules for use as labels and are directly coupled to proteins, polypeptides, other labels, nucleic acids and the like. The novel dye systems can be utilized for the quantitation of analytes in fluids, and in particular, in biological fluids. The novel dye systems can be tuned to specific exciting and emitting wavelengths so that low current sources, such as light emitting diodes and laser diodes, and detectors, such as photo diodes, can be used in the manufacture of fluorometers which can be battery powered and portable, for use, for example, in immunoassays dedicated to near-patient diagnostics.

### DISCLOSURE OF THE INVENTION

This invention relates to novel water soluble fluorescent dyes. These dyes can be tuned to specific excitation and emission wavelengths to accommodate a wide variety of assay or visualization systems.

Many novel hybrid phthalocyanine derivatives are disclosed and claimed.

Water soluble hybrid phthalocyanine derivatives are disclosed having (1) at least one donor subunit with a desired excitation peak; and (2) at least one acceptor subunit with a desired emission peak, wherein said derivative(s) is/are capable of intramolecular energy transfer from said donor subunit to said acceptor subunit. Such derivatives also may contain an electron transfer subunit. Axial ligands may be covalently bound to the metals contained in the hybrid phthalocyanine derivatives. The axial ligands of the dyes can be further elaborated with drug analogues and compounds, proteins, polypeptides and nucleic acids. Numerous compounds capable of intramolecular energy transfer as well as compounds for fluorescence energy transfer are claimed.

### BRIEF DESCRIPTION OF THE DRAWINGS

Fig. 1 depicts the structures of phthalocyanine, naphthalocyanine and anthranylocyanine.

Fig. 2 depicts the structures of silicon phthalocyanine, silicon naphthalocyanine and silicon anthranylocyanine.

Fig. 3 depicts the spectra of silicon phthalocyanine dihydroxide and the spectra of silicon 2,3-naphthalocyanine dihydroxide.

Fig. 4 depicts the general structure of ethenyl-substituted dipyrrometheneboron difluoro dyes.

Fig. 5 depicts the attenuation of the background signal as a function of increasing wavelength. The data was measured using a device as described in Applicant's allowed US Patent No. 5.458.852 filed May 21, 1992 entitled "Diagnostic Devices and Apparatus for the Controlled Movements of Reagents Without Membranes.

Fig. 6 depicts naphthalocyanine derivatives which emit in the near infrared.

Fig. 7 depicts general structures of fluorescent energy transfer naphthalocyanine compounds.

Fig. 8 depicts the absorbance spectrum of human serum between 200 nm and 1000 nm.

Fig. 9 depicts the structure of a novel hybrid phthalocyanine derivative, Silicon [di(1,6-diphenylnaphthalocyanine)] diphthalocyanine bis(dimethylhexylvinylsilyloxide).

Fig. 10 depicts the spectrum of Silicon [di(1,6-diphenylnaphthalocyanine)] diphthalocyanine bis (dimethylhexylvinylsilyloxide).

### MODES FOR CARRYING OUT THE INVENTION

This invention describes novel fluorescent molecules and diagnostic methods for their use. Developing a method for the visualization of a cellular component or a cell or for an assay which utilizes a fluorescent dye and which quantifies an analyte in a sample requires the use of a fluorometer. The fluorescent label, the sample and the instrument must be compatible with each other to achieve an accurate measurement. Several criteria for a fluorescent label as they relate to the sample and instrument are described below. First, the absorption or excitation and emission wavelengths of the dye should not correspond so closely to the absorption or fluorescence of the specimen or sample such that the sample affects the fluorescence measurement of the dye. Second, the Stokes shift of the dye should be as large as possible to minimize the measurement of background from the excitation wavelength. Third, the dye must be compatible with the fluid phase of the assay; that is, the dye must be water soluble. Fourth, the dye should be as bright as is necessary to achieve the desired sensitivity. Brightness is the product of the extinction coefficient and the quantum yield of the dye. Fifth, the instrument used to detect the fluorescent signal is generally designed around the specifications of the dye and the specimen or sample being visualized or assayed.

These points will be discussed in more detail and illustrate some of the intricacies in developing a fluorescent visualization technique or an assay using fluorescent dyes. One is limited either to dyes which have been synthesized or ones which must be synthesized in order to meet the above criteria. Using prior art methods, a very limited range of excitation and emission wavelengths can be planned for a specific molecule. The teachings of this invention allow one to prepare fluorescent dyes and labels which can be tuned to many excitation and emission wavelengths allowing for large Stokes shifts. Thus, designing a dye system with the specifications of the sample or specimen and the instrument is possible from the teachings of this invention, as opposed to the prior art methods which involve designing the instrument around the specifications of the dye. Tuning the dye system to accommodate the characteristics of the sample and the instrument results in an improved visualization process for the assay.

The excitation and emission wavelengths of the dye should not correspond to those of the sample being assayed or visualized, otherwise the sample can interfere with the measurement of the fluorescent signal. When absorption or emission wavelengths of the sample do correspond to those of the dye, in practice, one dilutes, for example, a serum or blood sample so that the interference by the sample is reduced or the interfering sample is washed away from the detection area. Indeed, currently, there is no fluorescent assay system on the market for the measurement of analytes in neat biological fluids, particularly blood, plasma or serum. One reason for the lack of fluorescent assay systems which detect analytes in neat samples is that no good fluorescent dye exists which meets all the criteria listed above, particularly for measuring fluorescence in biological samples. When the sample absorbs significantly at the excitation wavelength the amount of light which excites the sample is thus affected by the variation in the sample characteristics. For example, serum, plasma, or blood from different individuals will be different in their relative absorptivities, which differences translate into different intensities of excitation light used to excite the fluorescent label. The fluorescence emission of the dye is directly proportional to the intensity of the incident light, such that when the sample absorbs a portion of the incident light, the intensity of the fluorescent signal will vary accordingly. This results in measuring an incorrect or effected fluorescence emission. In addition, the emission wavelength of the dye should not correlate with the emission or absorbance of the sample because the sample will increase the measured fluorescence of the dye or the sample will absorb all or a portion of the dye fluorescence and also result in an incorrect or effected fluorescence emission. These problems are avoided when the sample is invisible to the excitation and emission wavelengths.

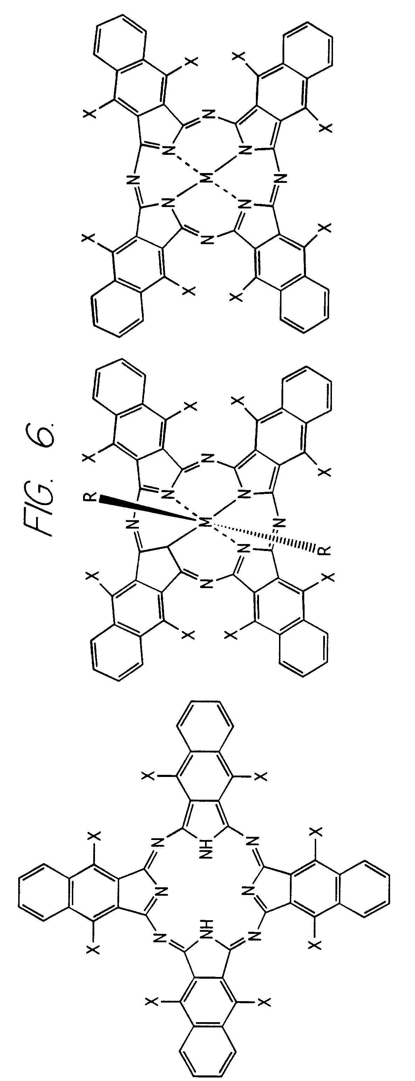

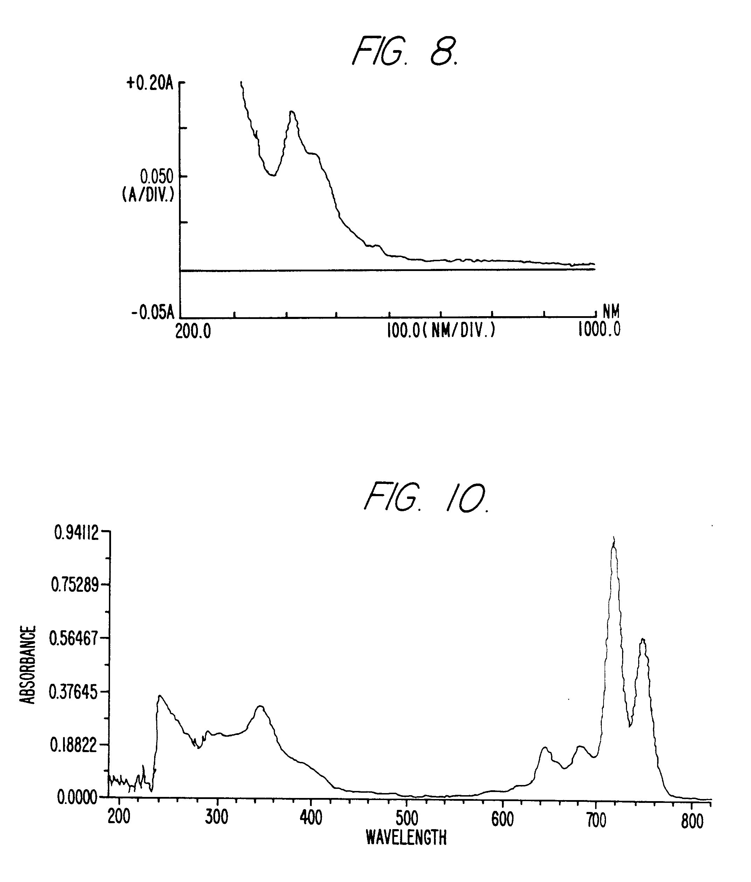

Figure 8 shows the spectrum between 200 nm and 1000 nm of human serum. Wavelengths above 600 nm absorb considerably less than those between 200 nm and 600 nm. Thus, both the absorption of the incident light and the effect on the fluorescence of a dye are minimal when exciting above 600 nm. Preferred excitation wavelengths for biological fluids, including urine, blood, serum or plasma is 600 nm or greater. Particularly preferred excitation wavelengths above 600 nm are those which correspond to the maximum light output of laser diodes and light emitting diodes. Preferred emission wavelengths are those above 600 nm. The intrinsic sample fluorescence can cause a high background signal if the emission wavelength of the dye and the sample are overlapping. In addition, the scattered light of the excitation source can also contribute to the background signal. The contribution of scattered light to the background can be seen, for example, in Figure 5. In general, the magnitude of the scatter is inversely proportional to the fourth power of the measured wavelength. This teaches that desired emission wavelengths are in the near-infrared or in the infrared region of the spectrum. The inventive teachings described herein provide for dyes and dye systems which excite above 600 nm and which emit above 650 nm and more preferred, above 730 nm.

The Stokes shift of the dye should be as large as possible to minimize the measurement of background from the excitation source so that the signal-to-background ratio at the limit of sensitivity is maximized. A large Stokes shift, however, will only maximize the efficiency of the fluorescence measurement and may not always result in an accurate fluorescence measurement. For example, table 3 shows data from several dye systems which were excited between 420 nm and 670 nm in either buffer, undiluted human serum and blood. The fluorescence intensity of the first dye system (line 1, table 1), when excited at 475 nm in serum and blood, is only 7.6% and 13%, respectively, of the intensity in buffer even though the Stokes shift is 205 nm. The second dye system (line 4, table 1), excited at 420 nm, is 28% and 4% in serum and blood of the intensity in buffer, respectively, with a 260 nm Stokes shift. The third and fourth dye systems (line 60 and line 59, table 1), excited at 670 nm and 650 nm and with 110 nm and 130 nm Stokes shifts, respectively, have fluorescence intensities which are comparable in buffer and in serum. The fifth dye system (line 107, table 1), excited at 670 nm with a 90 nm Stokes shift, has fluorescence intensities which are also comparable in buffer, serum and blood. The sixth dye system, which is a hybrid phthalocyanine derivative (line 1, table 2), has comparable fluorescence intensities in buffer, serum and blood when excited at 646 nm with a Stokes shift of 114 nm. The data show that the fluorescence intensity is greatly affected when the excitation wavelength is within the range of the absorbance of the sample in which the measurement is made. The data also show that the magnitude of the Stokes shift does not have an influence on the accuracy of the measurement. These data are representative of other dyes and dye systems which are excited at a wavelength where the sample absorbs. The effect of the decreased fluorescence emission is not a result of the emission wavelength (that is, 680 nm or 780 nm) because the samples absorb minimally at 680 nm and 780 nm. One skilled in the art can appreciate, that with the inventive teachings described herein, the wavelengths for excitation and emission of a dye system should be a function more of the absorption and emission characteristics of the sample rather than selecting only a dye system with a large Stokes shift.

The availability of dyes with Stokes shifts greater than 100 nm is greatly limited, particularly when the excitation wavelength is greater than 600 nm. To further limit the usefulness of available dyes, the solubility of the dyes in aqueous samples can be a problem because most dyes with large Stokes shifts are water insoluble.

The problem of a dye possessing a small Stokes shift is usually overcome in the engineering of the fluorometer by the use of monochromators or expensive optics which filter out the light from the excitation source. However, to overcome the loss in light intensity due to the filters, for example, one requires the use of high powered light sources. These light sources produce heat which must be dissipated in an instrument by using heat sinks or fans. The complexity of the fluorescence measuring device, both from an optical and a mechanical perspective, is thus greatly affected by the inadequacies of the dye system. With the advent of near-patient testing in hospitals and emergency departments, instruments which measure fluorescence in immunoassays will be required to be portable and uncomplicated to the technician. Thus, the future state of the art for the manufacture of, for example, fluorometers which are employed for immunoassays will be required to change to simple and portable instruments. The high powered light sources and expensive optics currently incorporated into fluorometers will not meet the requirements for small, portable instruments.

The instant invention teaches that fluorescent labels can be prepared with large Stokes shifts and be tuned to wavelengths both of which are compatible with excitation sources and emission detectors and which are compatible with the absorption and emission of the sample, for example, blood, serum, plasma, urine, ground water, and the like. The excitation and emission wavelengths of the novel fluorescent dyes and particles can generally be varied independently of each other.

The dye must be compatible with the fluid phase of the assay, or in other words, the dye must be water soluble. Many fluorescent dyes are water insoluble or poorly water soluble and these dyes are not easily used for labeling molecules, proteins, nucleic acids or cells. One skilled in the art will recognize that water insoluble dyes can be incorporated into latex particles as described in U.S. Patents 4,326,008, 4,609,689 and 5,154,887. Thus, water insoluble dyes can be made useful by incorporation into latex particles for visualization in a variety of assay formats.

The dye should be as bright as is necessary to achieve the desired sensitivity. If one knows the extinction coefficient and the quantum yield of the dye and the concentration of the target to be measured, it can be estimated whether the dye is bright enough to achieve the desired sensitivity. Incorporation of dyes into latex particles or the utilization of an enzyme which catalyzes the production of a fluorescent substrate are examples of techniques which one skilled in the art uses as amplification systems.

The instrument used to detect the fluorescent signal is generally designed around the specifications of the dye and the specimen or sample being visualized or assayed because of the limited numbers of dyes which can be successfully used. As discussed above, the components of the instrument are selected for a particular dye system since a useful instrument must be highly tuned to eliminate the light from the excitation source.

Each of the conditions described above impose limitations on dye systems which can be employed for measuring sub-picomolar concentrations of analytes, particularly in biological fluids. The limitations also impose restrictions on the design of an instrument to measure the fluorescence. The novel teachings of the instant invention allow the design, synthesis and tuning of dye systems to match, generally, nearly any instrument design.

An inventive teaching is described for tuning excitation and emission wavelengths of dyes so that the excitation and emission are compatible with the sample matrix in which the fluorescence is measured and the instrument for quantifying the fluorescence.

The teaching is to use hybrids of phthalocyanines, naphthalocyanines, anthranylocyanines (collectively termed hybrid phthalocyanine derivatives) and various derivatives of these classes of compounds which have different subunits depending on the desired excitation or emission wavelengths. The hybrid phthalocyanine derivatives are synthesized as water soluble compounds to be used for direct attachment to proteins, polypeptides other labels or nucleic acids. One advantage of hybrid phthalocyanine derivatives is that they allow one to create dyes and dye systems which have greater Stokes shifts with higher extinction coefficients at the excitation wavelength. This is accomplished by properly selecting the subunits which are to be tetramerized to form the hybrid phthalocyanine derivative structure and which will absorb the light at the excitation wavelength.

The selection of dye pairs is based on their ability to exhibit energy transfer (singlet-singlet energy transfer) at the appropriate excitation wavelength of the donor dye and the emission of the acceptor. Fluorescence energy transfer of two molecules is well known to those skilled in the art and the rate of energy transfer is described by Förster in Ann. Physik. (1948) 2, 55-75. Fluorescence energy transfer has been used as a spectroscopic ruler to predict proximity relationships in proteins, RNA and peptides (Annual Review of Biochemistry (1978), 47, 819-846) and also to probe geometrical details in particles (Physical Review Letters (1988) 61, 641-644). U.S. Patent 5,326,692 describes fluorescent particles with controllable enhanced Stokes shifts. U.S. Patents 4,542,104 and 4,666,862 describe fluorescence energy transfer in phycobiliproteins. These dye complexes are described for use as labels in immunoassays. The limited use, however, of phycobiliproteins and the expense of these natural protein complexes make them undesirable for use on a commercial scale. Some water soluble symmetrical tetraazaporphine derivatives have been described, for example, in EP-A-597389. Also, some unsymmetrical or hybrid phthalocyanines have been described, for example, in J. Am. Chem. Soc. 1990, 112, 9640-9641, Chemistry Letters 1992, 2031-2034 and Inorg. Chem. 1994, 33, 1735-1740, but this invention greatly expands the compounds which can be synthesized for use in immunodiagnostics to achieve adequate fluorescence intensities and desired excitation and emission characteristics. The ratio of the various diiminoisoindiline or dicarbonitrile precursors and their substitution by electron donating or electron withdrawing groups in the synthesis of the hybrid phthalocyanines, naphthalocyanines and anthranylocyanines will affect the absorption spectrum and the excitation and emission wavelengths of the compounds. This is taught and applied to the novel dyes herein.

The selection of the dye molecules should be related to the sample to be analyzed and the instrument for measuring the fluorescence. For example, when developing an assay for an analyte in a biological medium, such as blood, serum or a cell extract, the intrinsic absorbance and fluorescence of the sample must be considered. Serum and cellular components absorb in the ultraviolet spectrum as well as in the visible spectrum up to around 600 nm and the intrinsic fluorescence can broadly approach 600 nm. In addition, samples which contain small particles, such as dirt particles in ground water, lipoproteins in serum or blood, cells and cellular particles and components will scatter the excitation light which results in a higher background signal. The ideal dye couple would include the donor dye which would be excited or absorb at above 600 nm and emit at a wavelength which the acceptor dye absorbs, and the acceptor dye should emit at a wavelength above 600 nm. In the case of a single dye system, for example, with the use of hybrid phthalocyanine derivatives, the excitation and emission wavelengths should also be above 600 nm. The sample, for example, serum, then does not affect fluorescence of the acceptor dye because the sample poorly absorbs at the absorption of the donor dye and the acceptor dye emits at a wavelength where the sample does not absorb or fluoresce.

Fluorescent dye molecules will exhibit fluorescence quenching because of the close proximity of the dyes to each other. When using dyes one must optimize the concentration of dye as it relates to quenching. In a situation where more than one acceptor dye is used to minimize fluorescence quenching and to maximize fluorescence intensity, one may use different acceptor dyes which have emission peaks which are within about 25 nanometers of one another. The emission of both acceptor dyes may be useful if the fluorometer is set-up to measure a wide band pass of fluorescence, for example, about a 20 to 60 nm bandpass.

Another important consideration is the efficiency of the fluorescence energy transfer. In practice, if the energy transfer efficiency is not close to 100%, then one can observe the fluorescence of the donor dye. The emissions of the donor and acceptor wavelengths can overlap partially with each other when efficient energy transfer is not obtained and complicate the selection of filters for use in a fluorometer. The decrease in.the energy transfer efficiency can also be directly related to a decrease in the emission of the acceptor dye, resulting in a system which may not be as bright as one with efficient energy transfer. In addition, under conditions of inefficient energy transfer, slight changes in the sample or in solution conditions, for example, pH and ionic strength, may affect the magnitude of energy transfer efficiency and thereby may affect the intensity of the fluorescent signal.

Although a particular pair of dyes has acceptable overlapping excitation and emission wavelengths (for example, see Proc. Natl. Acad. Sci. USA 1969, 63, 23-30), they may have suboptimal (less than 80%) efficiency of energy transfer. The process to determine whether 2 or more dyes will exhibit efficient energy transfer is through experimentation after the appropriate spectral overlap criteria are met. The measured fluorescence at the donor dye emission wavelength divided by the fluorescence of the donor dye is the efficiency of fluorescence energy transfer. Ideally, in practice, the emission of the donor dye should be undetectable or only slightly detectable so that the effective Stokes shift is not reduced because of the donor dye emission. Preferred fluorescence energy transfer efficiencies are 80% or greater and particularly preferred fluorescence energy transfer efficiencies are 90% or greater.

In yet another aspect of this invention, the synthesis of phthalocyanine derivatives and hybrid phthalocyanine derivatives with axial ligands reduces the stacking of the aromatic ring system, thus minimizing the interactions between molecules and maximizing fluorescence intensities.

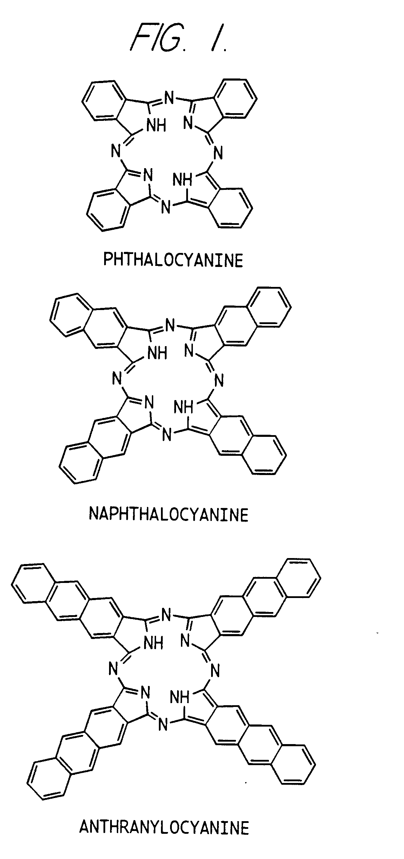

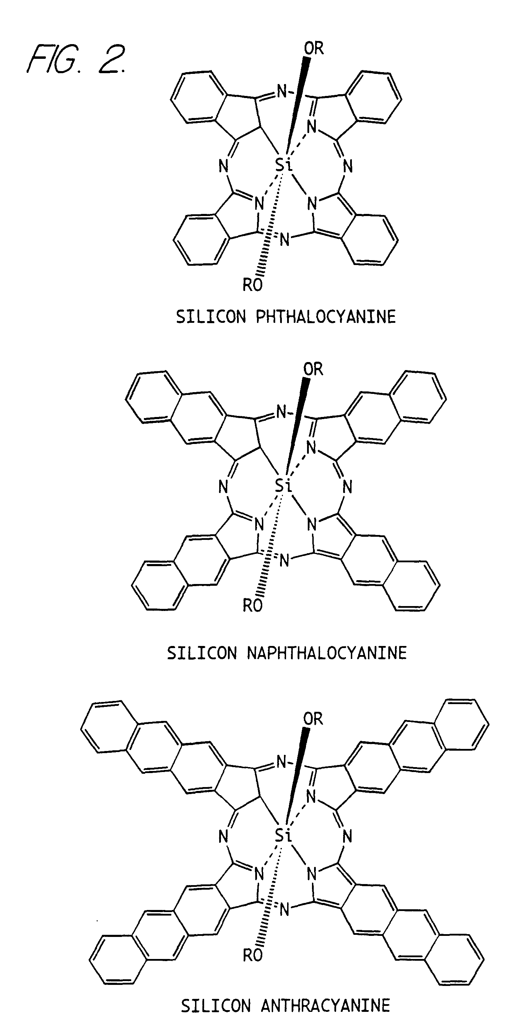

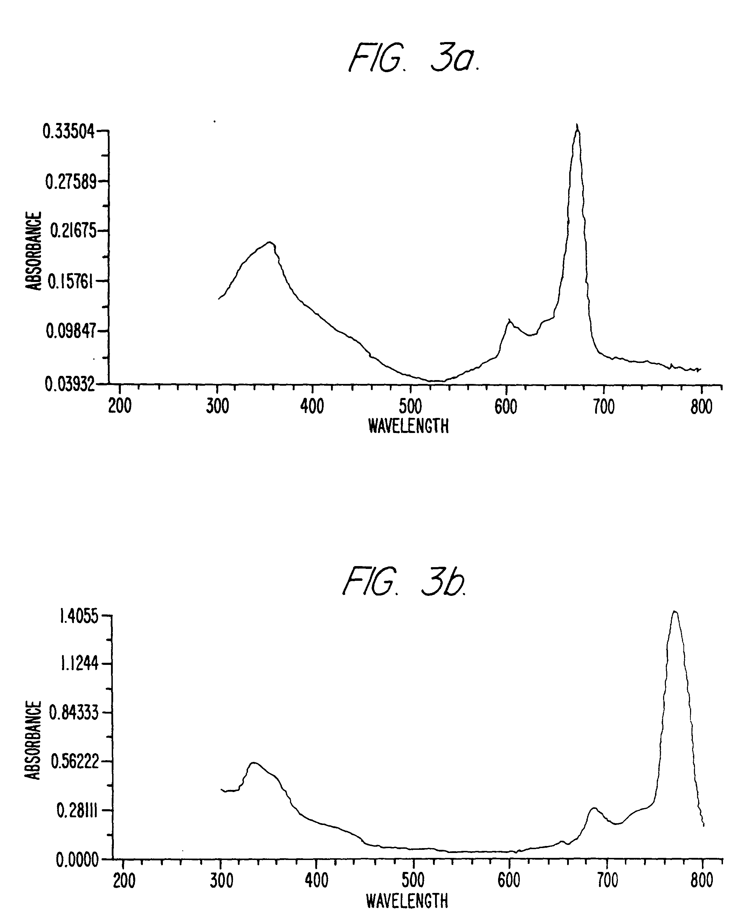

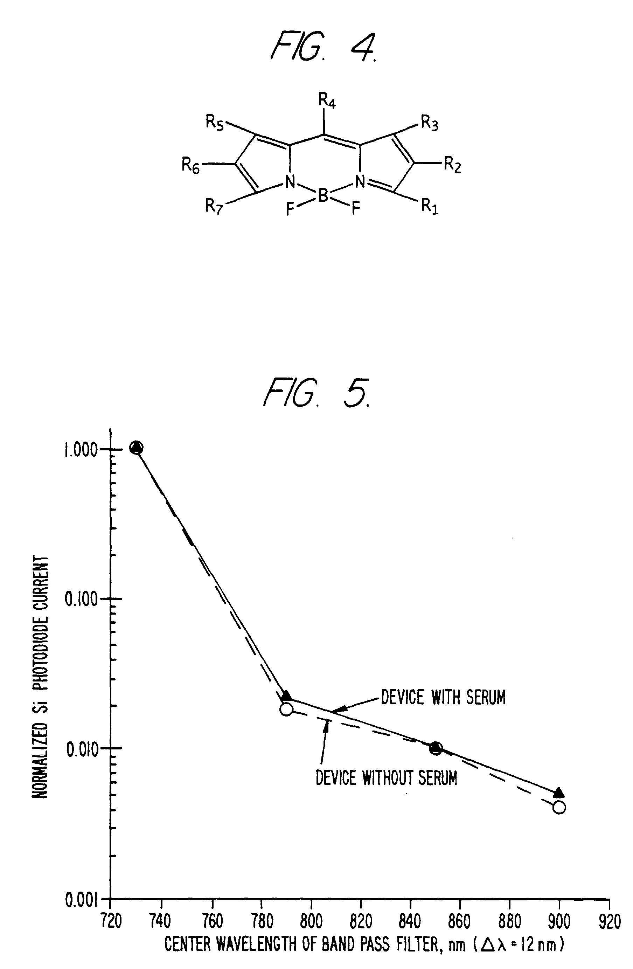

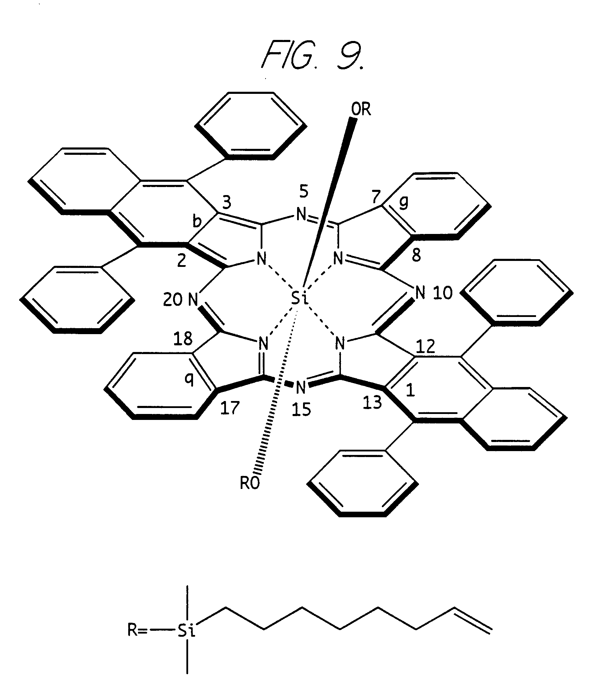

Figure 1 shows preferred acceptor dyes which are phthalocyanines, naphthalocyanines and anthranylocyanines. Figure 2 shows particularly preferred acceptor dyes which are derivatives of silicon phthalocyanines, naphthalocyanines and anthranylocyanines, where R is hydrogen or an alkylcarbon chain from 1-20 carbons, either saturated or unsaturated, having 0-10 heteroatoms (N,O,S), and having 0 or 1 siloxide groups. The best mode compounds are those in which R =
Si(CH₃)₂C₆F₅
Si(C₆H₁₃)₃
Si(CH₃)₂(CH₂)₃CN
Si(CH₃)₂(CH₂)₁₀COOCH₃
Si(CH₃)₂CH=CH₂
Si(CH₃)₂(CH₂)₁₀COOH
Si(CH₃)₂(CH₂)₄Cl; and
Si(CH₃)₂(CH₂)₆CH=CH₂.
The parent compounds of the phthalocyanines and naphthalocyanines are preferred. Also preferred parent compounds are the anthranylocyanines which have emissions around 850 to 900 nm. These three classes of preferred parent compounds will collectively be called "phthalocyanine derivatives" and may or may not have an included metal and may or may not have axial ligands. Also, preferred parent compounds include "hybrid phthalocyanine derivatives" which have 2 or more different subunits of the 4 total subunits and may or may not have an included metal and may or may not have axial ligands. An example of a hybrid phthalocyanine derivative containing a metal and an axial ligand is illustrated in Figure 9. The emission wavelengths for the phthalocyanine derivatives or the hybrid phthalocyanine derivatives are particularly useful for quantifying fluorescence in biological samples and tissues and for minimizing the background scatter intensity. Those skilled in the art can appreciate that phthalocyanine derivatives and hybrid phthalocyanine derivatives can be synthesized, for example, by derivatization of the phenyl, naphthyl or anthranyl rings with various substitutes to yield different molecules. These variants are within the scope of the instant invention. Derivatives of tetraazaporphine are also within the scope of the instant invention. The derivatization of the aromatic structure of phthalocyanine derivatives and hybrid phthalocyanine derivatives can produce blue or red shifted excitation or emission wavelengths. The choice of the donor dye to excite the phthalocyanine or hybrid phthalocyanine derivatives is dependent on having a donor dye emission wavelength which corresponds to the appropriate range of absorbance wavelengths of the phthalocyanine or hybrid phthalocyanine derivative. Figure 3 shows the absorbance spectra of the silicon dihydroxyphthalocyanine and silicon dihydroxynaphthalocyanine in dimethylformamide. A potential range of excitation of the these acceptor dyes by the donor dye is between approximately 550 nm and 670 nm and 600 nm and 760 nm, respectively. One skilled in the art will recognize that many dyes would be candidates for the donor dye because of the wide useful range of wavelengths which can excite the acceptor dyes. Indeed, the phthalocyanine derivative can be the donor for the naphthalocyanine derivative. The choice of the acceptor dye should meet the criteria outlined above. Several examples are described which illustrate the versatility of this novel approach.

If one wants to build an instrument with an excitation source which has a maximum intensity at 480 nm and a detector which has a good quantum efficiency at 600 to 700 nm, the donor dye should be capable of being excited at 480 nm. Assuming that a phthalocyanine derivative is the acceptor dye for emission at 680 nm, the donor should then emit in the range of 550 to 670 nm.

Preferred classes of dyes for this application are styryl, phenylbutadienyl and phenylhexatrienyl dyes. Styryl dyes are those of the following formula: 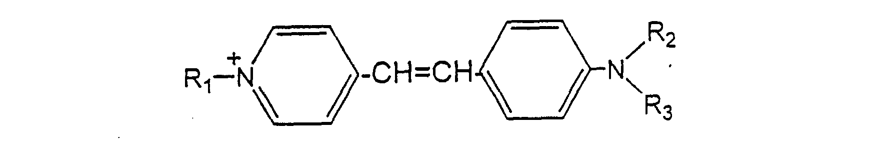 and phenylbutadienyl dyes are of the formula: 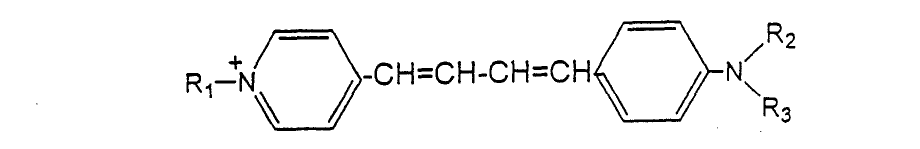 and phenylhexatrienyl dyes are of the formula: 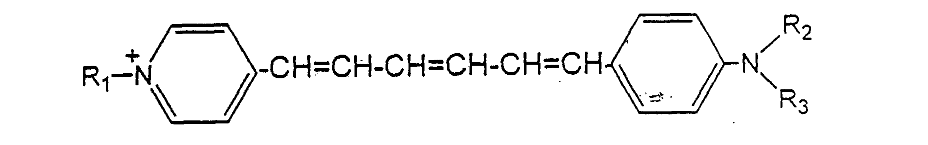 wherein R1, R2 and R3 can be the same or different and R1, R2 and R3 are H or alkylcarbon chains from 1-20 carbons, either saturated or unsaturated, and having 0-10 heteroatoms (N, O, S) .

In general, these dye classes excite approximately between about 470 and 530 nm and emit approximately between 600 and 780 nm (see Molecular Probes Handbook of Fluorescent Probes and Research Chemicals by Richard P. Haugland, 1992-1994, p. 156). A particularly preferred styryl dye is the trans-4-[4-(dibutylamino)styryl]-1-methylpyridinium iodide (Aldrich Chemical Co.) which has its maximum absorbance at 486 nm in dimethylformamide and its emission at 600 nm. One skilled in the art will recognize that the substituents off the aniline nitrogen and the pyridium nitrogen of these classes of dyes can vary.

In another application, an instrument system is built which has a source of maximum intensity at 420 nm and a detector as described in the above example. The dye system here can include the phthalocyanine acceptor; however, a different donor must be employed. A preferred donor for this application is a meso-tetra-2-aminophenylporphine (Porphyrin Products, Inc., Logan UT) which has a maximum absorbance for excitation at 418 nm in dimethylsulfoxide and an emission around 655 nm.

In a particularly preferred application, an instrument system is built to perform immunoassays in neat blood or serum or in various biological specimens. The excitation source is a light emitting diode (LED) or laser diode which has a maximum intensity around 650 nm to avoid absorption of the light by the blood or serum sample. The detector has good quantum efficiency at 700 to 800 nm so a preferred acceptor dye is a naphthalocyanine derivative which has an emission at approximately 780 nm, an emission wavelength which is generally not in common with blood or serum samples or biological specimens. A donor dye for the naphthalocyanine acceptor should absorb at around 650 nm to coincide with the source and emit between approximately 660 nm and 760 nm. Preferred classes of dyes for this donor application are the carbocyanine dyes and the ethenyl-substituted dipyrrometheneboron difluoro dyes, as described in U.S. Patent Nos. 5,187,288, 5,248,782 and 5,274,113.

In yet another particularly preferred application, an instrument system is built to perform immunoassays in neat blood, plasma or serum or in various biological specimens. The excitation source is an LED or a laser diode which has its maximum intensity around 670 nm to avoid absorption of the light by the blood, plasma or serum sample. The detector has good quantum efficiency at 700 to 800 nm so preferred acceptor dyes are silicon [(diphthalocyanine)dinaphthalocyanine] ligands or a naphthalocyanine derivative which have an emissions at approximately 760 nm and 780 nm, respectively, emission wavelengths which are generally not in common with blood or serum samples or biological specimens. A donor dye for the preferred acceptors should absorb at around 670 nm to coincide with the source and emit between approximately 660 nm and 760 nm. Preferred donor dyes are silicon phthalocyanine with axial ligands.

In yet another particularly preferred application, for immunoassays in neat blood or serum, the excitation source is around 790 nm and the emission wavelength is around 900 nm. A preferred dye for a single dye system is a silicon 1,6-octaethoxynaphthalocyanine bis(dimethylhexylvinylsilyloxide) which is excited at 790 nm and emits at about 900 nm.

Preferred dyes for use as donor dyes for naphthalocyanines and naphthalocyanine derivatives are, carbocyanines and ethenyl-substituted dipyrrometheneboron difluoro dyes, as described in U.S. Patent Nos. 5,187,288, 5,248,782 and 5,274,113 which have excitation wavelengths up to 790 nm and emission wavelengths between about 670 nm and 800 nm.

Preferred carbocyanine dyes, which generally excite between 500 and 750 nm (see Molecular Probes Handbook) are of the general formula: 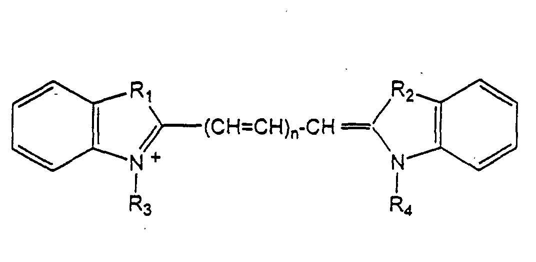 wherein n is 1 or 2; or 3; wherein R1 and R2 are S, N, or O; and wherein R3 and R4 are H or alkylcarbon chains of from 1-20 carbons, either saturated or unsaturated and having 0-10 heteroatoms (N, O, S).

Also preferred carbocyanine dyes are also of the general formula: 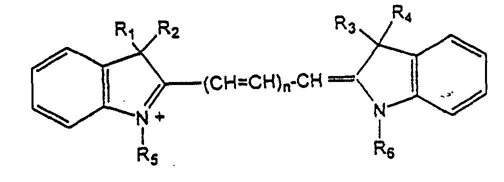 wherein n is 1 or 2; or 3; wherein R1-R6 are H or alkylcarbon chains of from 1-20 carbons, either saturated or unsaturated and having 0-10 heteroatoms (N, O, S).

Preferred donor dyes are also the ethenyl-substituted dipyrrometheneboron difluoro dyes, which generally excite above 500 nm (see Molecular Probes Handbook) and are of the general formula as depicted in Fig. 4, wherein R1-R7 include substituents as described in U.S. Patent Nos. 5,187,288, 5,248,782 and 5,274,113.

Particularly preferred donor dyes are 1,1'-dihexyl 3,3,3',3'-tetramethylindocarbocyanine iodide, 1,1'-diethyl 3,3,3',3'-tetramethylindodicarbocyanine iodide and (E,E)-3,5-bis-(4-phenyl-1,3-butadienyl)-4,4-difluoro-4-bora-3a, 4a-diazo-5-indacene (from Molecular Probes Inc., Eugene, OR), which have absorption maximums of 642 nm, and 645 nm and 650 nm and emission maximums of 674 nm and 665 nm, and 670 nm, respectively, in dimethylformamide. These particularly preferred dyes together with a naphthalocyanine derivative will excite with a 650 nm source and emit at approximately between 780 nm and 870 nm. One skilled in the art will recognize that the excitation and emission spectra for any particular dye has a Gaussian form and therefore the excitation source does not need to correspond exactly to the excitation maximum of the donor dye in order to obtain an intense fluorescent signal. Likewise, the donor emission does not have to coincide with the highest absorption of the acceptor dye in order to achieve efficient energy transfer. One skilled in the art will also recognize that the substituents at and on the 1 and 3 positions of the carbocyanines and the substituents at the R1 and R7 positions of the dipyrrometheneboron difluoro dyes, and the conjugation between the ring structures can vary.

Also preferred emission wavelengths of fluorescent particles range from about 800 nm to 1000 nm. This near infra-red region is important because the scattering component of the light decreases substantially, thus lowering the background of the fluorescent measurement. In addition, biological samples do not absorb or fluoresce substantially in the 800 nm - 1000 nm range. Particulate materials in the samples, for example, lipoproteins in serum, particles in ground water, cellular debris in biological samples and the like, can increase the background signal because of scattered light and the measurement of the scattered light is minimized in the 800-1000 nm range.

Figure 5 illustrates the attenuation of the background signal as the wavelength of the measured light increases from 730 nm to 900 nm in an immunoassay device, as described in allowed App. Ser. No. 07/887,526 (which is herein incorporated by reference), containing either neat human serum or no serum. This figure shows that the background signal decreases by a factor of 5 when measuring at 900 nm as compared to 790 nm when the illumination source is a 1 milliwatt ("mW") 670 nm laser diode. In addition, excitation of neat serum at 670 nm does not result in a significant measurable fluorescence between 730 nm and 900 nm. Thus, for example, the signal to background ratio of the measurement of fluorescence of a dye which emits at around 900 nm as compared to a dye emitting at around 790 nm would be improved by a factor of 5. The signal to background ratio improves by a factor of about 30 when measuring emission at 780 nm as compared to 730 nm (see figure 5). Preferred dyes, for example as described in J. Chem. Soc. Perkin Trans. 1, (1988), 2453-2458, which emit above 780 nm include derivatives of the naphthalocyanine and anthranylocyanine classes (Fig. 1) and the naphthalocyanine class is characterized by the general formulae, as depicted in Fig. 6, where M is a metal such as Si, Ge, Al, Sn and Ti and the like, and where R is an axial ligand, alkyl or aryl with or without a silicon (preferred axial moieties are synthesized from alkyl or aryl silyl chlorides), and where X is an electron donating group or groups which can be the same or different, including, such as amino, hydroxyl, alkoxy, aryloxy, phenyl, and alkyl. The electron donating character of the X group or groups red-shifts the emission wavelength as compared to the general naphthalocyanine compounds (Figure 1).

For example, the compounds described in examples 26, 27 and 28 are illustrative of dyes which have emission wavelengths around 850 nm. These preferred dyes would yield an improved signal to background ratio as compared to dyes emitting at 780 nm (See Fig. 5). Electron withdrawing groups can also be utilized for the X groups, such as halogen, nitro, cyano, sulfate, carboxyl and carboxyalkyl, which will blue shift the excitation or emission wavelengths. Preferred donor dyes for this class of near infra-red emitting dyes are those which have emission wavelengths which correlate to the absorbance characteristics of the acceptor dye. Preferred dyes for this application are the ethenyl-substituted dipyrrometheneboron difluoride dyes, as described in U. S. Patent Nos. 5,187,288, 5,248,782 and 5,274,113.

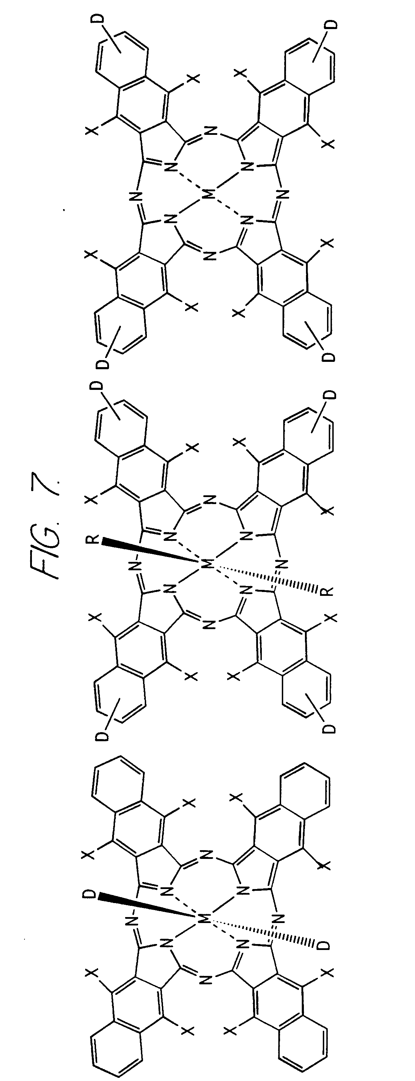

The geometrical orientation of the dipoles of the donor and acceptor dyes will affect the efficiency of energy transfer between them. The donor and acceptor dyes can be synthesized to form a compound of optimal dipole geometry, which, in solution, exhibits efficient fluorescence energy transfer ("FET"). Phthalocyanine derivatives can be utilized for this application for the acceptor moiety, where the phthalocyanine derivative can be substituted with electron donating or withdrawing groups (as described above) to accommodate the desired excitation and emission wavelength. For example, preferred naphthalocyanine compounds for this application are those as depicted in Fig. 7, where X is hydrogen or electron donating groups, such as amino, hydroxyl, alkoxy, aryloxy, phenyl, alkyl and D is the donor dye covalently attached to the naphthalocyanine derivative at a distance which allows for energy transfer between the donor and acceptor.

By applying the teachings of this invention, all phthalocyanine of hybrid phthalocyanine derivatives can function as donor or acceptor molecules. For example, a silicon ortho octaethoxy(phthalocyanine) derivative will emit at approximately 750 nm to 780 nm, similar to a silicon naphthalocyanine derivative. Generally, the distances between donor and acceptor are about 5 angstroms to 60 angstroms, and preferably from 5 angstroms to 15 angstroms. In addition, each naphthalocyanine derivative can have 1-4 donor dyes attached, depending on the required application of the FET compound. Suitable donor dyes are those which emit in the absorbance range of the acceptor dye. Example 29 describes the synthesis of a fluorescein-silicon phthalocyanine FET compound. One skilled in the art will appreciate that with the inventive teachings described herein, many FET compounds may be synthesized for many particular applications requiring specific excitation and emission wavelengths.

As used herein, the term "hybrid phthalocyanine derivatives" refers to all classes of hybrid phthalocyanines, naphthalocyanines and anthranylocyanines and their derivatives, with or without metal and axial ligands, including tetraazaporphines and their derivatives. The novel hybrid molecules described herein appear to exhibit intramolecular energy transfer. The hybrid phthalocyanine derivatives can be synthesized from diiminoisoindoline or derivatives of diiminoisoindolines and incorporate a metal, for example, silicon, and elaboration with axial ligands or they can be synthesized from dicarbonitrile derivatives of benzene, naphthalene or anthracene compounds, respectively, for subsequent inclusion of various metals and elaboration with axial ligands. Hybrid molecules comprised of derivatives of tetraazaporphines, as described in Inorg. Chem. (1994), 33, 1735-1740, are also within the scope of the hybrid phthalocyanine derivatives of the instant invention. A synthetic strategy for hybrid phthalocyanine derivatives with 2 different subunits is described, for example, in J. Am. Chem. Soc. (1990), 112, 9640-9641, Inorg. Chem. (1994),33, 1735-1740, Chem. Letters, (1992), 763-766, Chem. Letters, (1992); 1567-1570 and Chem. Letters, (1992), 2031-2034. These references describe the synthesis of hybrid molecules with zinc metal or without metal and without axial ligands. The character of the diiminoisoindoline and its derivatives will dictate the excitation and emission characteristics of the molecule. Moreover, incorporation of dyes with axial ligands, as taught herein, will result in minimum quenching and maximum fluorescence intensity.

Axial ligands are beneficial on water soluble compounds because the axial ligands will minimize interaction of the hybrid molecule with, for example, proteins, antibodies and nucleic acids, which may or may not be covalently coupled to the hybrid molecule. The axial ligand may itself, impart water solubility to the hybrid phthalocyanine derivative.

Examples of water soluble phthalocyanine derivatives are disclosed in Examples 92, 95-98, 108, 110, 114-124, and 126-128.

Novel hybrid phthalocyanine derivatives are described herein, which contain 3 or 4 different subunits, and allow for larger Stokes shifts. In these derivatives, excitation occurs with the subunit which has the highest energy or the lowest wavelength absorption and the emission occurs in the lowest energy subunit.

The desired excitation and emission wavelengths of the hybrid phthalocyanine derivative will determine the types of diiminoisoindoline derivative and dicarbonitrile derivative precursors which are used in the synthesis of the hybrid phthalocyanines. The desired excitation and emission wavelengths are generally dictated by the sample, the type of fluorescent measurement and the instrument. Various combinations of diiminoisoindoline derivative and dicarbonitrile derivative precursors also may be combined to form a hybrid phthalocyanine derivative which may have a red shifted or blue shifted excitation and/or emission wavelength pattern.

In general, electron donating substituents on the diiminoisoindoline or dicarbonitrile precursors, particularly situated at the ortho positions (that is, ortho to the tetraazaporphine structure as indicated in Figure 6 for the X substituents) of the phthalocyanine structure, such as amino, hydroxyl, alkoxy, aryloxy, phenyl, and alkyl, will red shift the excitation and/or emission wavelengths. Conversely, electron withdrawing substituents, also particularly at the ortho positions, such as halogen, nitro, cyano, sulfate, carboxyl and carboxyalkyl, will blue shift the excitation or emission wavelengths. In addition, positions on the subunits other than the ortho positions can affect the excitation and emission characteristics of the hybrid phthalocyanine derivative. The choice of either diiminoisoindoline or dicarbonitrile precursors for the synthesis of the hybrid phthalocyanine derivatives is related to the desired presence or absence of metal and the type of metal in the hybrid molecule. For example, when using the diiminoisoindoline precursors in the synthesis, a silicon metal can be incorporated during the tetramerization reaction to form the phthalocyanine derivative structure. The silicon can be further modified to a silicon dihydroxy phthalocyanine derivative molecule so that axial ligands can be elaborated with, for example, various silyl chloride reagents. The importance of axial ligands in reducing quenching and maximizing fluorescence intensity is evident for both phthalocyanine/naphthalocyanine molecules and the hybrid phthalocyanine derivatives (see example 65).

The axial ligands are also useful for further elaboration of the molecules, for example, for attaching another fluorescent molecule, for attaching to a ligand, protein, polypeptide or nucleic acid or for changing the charge of the molecule using sulfate, carboxylic acid or amino substituents which can affect solubility of the molecule. In the case of using axial ligands to attach the water soluble dye to ligands, proteins, polypeptides or nucleic acids, a mono- or bis- substituted metal can be utilized. The mono-substituted metal in the dye, however, yields only one axial ligand onto which the chemistry of attachment is made. The other face of the dye, after attachment to a ligand, protein, polypeptide or nucleic acid, which has no axial ligand, may interact with neighboring molecules (proteins, polypeptides, nucleic acids and result in quenching of fluorescence. The bis-substituted dye can minimize potential interactions between neighboring molecules when one axial ligand is used for attachment and the other is unattached. In this case, the unattached axial ligand can be synthesized such that the terminal atom of the unattached axial ligand imparts water solubility to the molecule, for example, a sulfate, carboxyl, or an amino derivative, such that interactions between neighboring molecules is minimized. In the case of utilizing water soluble hybrid phthalocyanine derivatives, for example, for competitive immunoassays, the ligand analogue of the target ligand which is being measured, can be attached to the dye through the axial ligand(s). The axial ligands of the water soluble phthalocyanine and hybrid phthalocyanine derivatives can also contain functional groups, for example, amines, carboxylic acids and esters, alkyl halides, thiols, and thio ester for attachment of ligands, proteins, polypeptides and nucleic acids. The axial ligands can also impart water solubility on the phthalocyanine and hybrid phthalocyanine derivatives when the axial ligand is comprised of poly(ethylene oxide). The carboxylic acid ester or the thioester groups on the axial ligands can be hydrolyzed in dilute base to the carboxylic acid and thiol groups, respectively. The chemical reactions to attach the axial ligands to ligands and ligand analogues, proteins, polypeptides and nucleic acids should be compatible with the functional groups of the compounds or macromolecules. For example, an amine on the axial ligand of the dye can be reacted with a compound or macromolecule containing a carboxylic acid or an alkyl halide, an alkyl halide on the axial ligand of the dye can be reacted with an amine or a thiol on the compound or macromolecule, a thiol on the axial ligand of the dye can be reacted with an alkyl halide or a maleimide group on the compound or macromolecule. Thus, compounds, such as ligands, ligand analogues and macromolecules, such as nucleic acids, polypeptides and antibodies can be reacted specifically to the dye by reaction with functional groups on the dye.

In general, phthalocyanine and hybrid phthalocyanine derivatives can be made water soluble by sulfonating the compounds using, for example, sulfuric acid or chlorosulfonic acid (see Gilbert, "Sulfonation and Related Reactions", Interscience, New York, 1965; Cerfontain, "Mechanistic Aspects in Aromatic Sulfonation and Desulfonation", Interscience, New York, 1968, *Int. J. Sulfur Chem.* C6, 123-136 (1971)) The sulfonation of the aromatic ring structure of the dye molecules can occur at various carbons of the ring. Added water solubility of the dye molecules can be achieved using axial ligands comprised of poly (ethylene oxide).

When using the dicarbonitrile precursors, the phthalocyanine derivative is synthesized without metal, but various metals can subsequently be included, for example, Ge, Al, Sn, Ti and the like. These metals can also be elaborated with axial ligand(s), depending on the valence of the metal.

The tetramerization reactions of the diiminoisoindoline or dicarbonitrile precursors to form the hybrid phthalocyanine derivatives can be directed so that opposing subunits can be the same. This is accomplished, for example, with the use of bulky substituents on the precursors so that in the tetramerization reaction, like subunits with bulky substituents cannot be adjacent because of steric considerations. Bulky phenyl substituents have been used on dicarbonitrile precursors to direct the precursors tetramerization to be opposing subunits as described in Inorg. Chem. (1994),33, 1735-1740, Chemistry Letters (1992), 2031-2034 and Chemistry Letters (1992),1567-1570.

Preferred hybrid phthalocyanine derivatives have similar opposing subunits so that two different subunits comprise the structure. Particularly preferred hybrid phthalocyanine derivatives have similar opposing subunits on one axis and different opposing subunits on the other axis. The nature of the particularly preferred molecules is that red or blue shifted excitation or emission wavelengths and a longer Stokes shift can result because of the selection of the precursor molecules for the tetramerization reaction. For particularly preferred hybrid phthalocyanine derivatives, for example, the "donor" diphenyldiiminoisoindoline or the diiminoisoindoline precursors would contribute to 650 nm absorbance of the hybrid molecule, and thereby to the excitation of the hybrid molecule. The diphenyl phenyldiiminoisoindoline or the phenyldiiminoisoindoline precursors would act as an "electron transfer subunit" to the "acceptor subunit", which would be a dialkoxy or aryloxy phenyldiiminoisoindoline precursors, so that emission is dictated at the lowest energy by the acceptor subunit at about 850 nm. The nature of the "electron transfer subunit" is important because it is not desirable for this subunit to emit because then the desired emission of the acceptor subunit will not take place. Thus, the highest occupied molecular orbital (HOMO) and lowest unoccupied molecular orbital (LUMO) character of the electron transfer subunit should be designed with reference to the donor and acceptor subunit molecules. The relationship of the energies of the HOMO and LUMO as they relate to excitation and emission are taught by Pariser et al., J. Chem. Phys. (1953), 21, 767-776, by Pople, Trans. Faraday Soc. (1953), 49, 1375-1385, by McHugh et al., Theoret. Chim. Acta (Berlin) (1972), 24, 346-370 and by Kobayashi et al, Inorg. Chem. (1994), 33, 1735-1740, Chemistry Letters (1992), 2031-2041, Konami et al., Molecular Physics (1993), 80, 153-160.

Another application requires the hybrid molecule to have two excitation wavelengths, one at approximately 650 nm and another at about 680 nm with emission for both excitations at about 760 nm. Thus, the precursors responsible for the excitation could be a diiminoisoindoline for the 650 nm and a tetrafluorodiiminoisoindoline for the 680 nm excitations. The emitting subunit, which can also be used to direct the tetramerization reaction so that the emitting subunits are opposed in the molecule, can be a diphenyl phenyldiiminoisoindoline. The excitation and emission wavelengths of the resulting hybrid phthalocyanine derivative are thus generally representative of the individual diiminoisoindoline precursors.

Yet another application requires excitation at about 650 nm and emission at about 750 nm. The precursors responsible for excitation and emission could be diiminoisoindoline and diphenyl phenyldiiminoisoindoline, respectively. The latter precursor also acts to direct the emitting subunits to be opposed.

In another application, a large extinction coefficient at the excitation wavelength is desired for excitation at about 650 nm. The emission wavelength should be at about 850 nm. The precursors responsible for excitation could be a diphenyldiiminoisoindoline, which would direct these subunits to be opposed and thereby two subunits would contribute to provide the desired extinction coefficient. A phenyldiiminoisoindoline derivative precursor could act as an electron transfer subunit and an alkoxyphenyldiiminoisoindoline precursor could be the acceptor with a characteristic emission at about 850 nm.

In another application, two emission wavelengths are desired from a compound which is excited at a single wavelength. The desired excitation is around 650 nm and the emission should be around 760 nm and 810 nm. The precursor responsible for excitation could be a tetrafluorodiiminoisoindoline or a tetrafluorobenzene-1,2-dicarbonitrile. The precursor responsible for emission could be a dibutoxy-phenyldiiminoisoindoline or a 3,4-dibutoxy-naphthalene-1,2-dicarbonitrile, respectively.

### Use of Water Soluble Hybrid Phthalocyanine Derivatives in Assays

Water soluble hybrid phthalocyanine derivatives can be attached to antibodies for use in non-competitive immunoassays or ligand analogues for use in competitive immunoassays in reaction mixtures of the assays. In the case of non-competitive assays, the reaction mixture would include at least one target ligand and at least one water soluble hybrid phthalocyanine derivative having attached thereto at least one receptor specific for target ligand, forming an antibody (fluorescent) conjugate. In the case of competitive assays, the reaction mixture will include at least one target ligand, at least one receptor specific to the target ligand, and at least one water soluble hybrid phthalocyanine derivative having attached thereto at least one ligand analogue, forming a ligand analogue (fluorescent) conjugate. In addition, in certain embodiments, the antibody conjugates and ligand analogue conjugates can be utilized as non-fluorescent labels. The non-fluorescent labels would be used in applications where only a color response, measured by reflectance in an assay device, is necessary.

The fluorescent conjugates of water soluble-hybrid phthalocyanine derivatives, which are smaller in molecular weight than the fluorescent particles described herein, will diffuse faster in solution and result in binding reactions which have faster kinetics. Fast kinetics of the binding reactions in assays are preferred because the assays will reach equilibrium binding in a shorter time, and in turn, assay results can be obtained in a shorter time. The antibody conjugates bound to target ligands in the non-competitive reaction mixture and the ligand analogue conjugates not bound by receptors specific to the target ligands in the competitive reaction mixture can be bound to a solid phase consisting of receptors specific to another epitope of the target ligand of the target ligand-antibody conjugate complexes and of receptors specific to ligand analogues of the ligand analogue conjugates, respectively. The fluorescent conjugates unbound by the solid phase are removed and the fluorescence (or color) of the bound conjugates is measured. The measured fluorescence (or color) is related to the target ligand concentration.

In the case of quantifying nucleic acids in samples, the novel compounds described in the instant invention are useful because of their brightness and because of the near infrared emission characteristics. In general, in designing an assay for a nucleic acid, one selects a probe molecule which is complementary to the nucleic acid to be quantified. The probe molecule is then labeled, generally covalently, with a signal generator. The signal generator can be a water soluble phthalocyanine derivative or hybrid phthalocyanine derivative. The labeled probe molecule is then introduced into a biological sample suspected of containing the target nucleic acid, and the labeled probe sequence assembles with the target nucleic acid. The labeled probe/target nucleic acid can then be immobilized onto a surface which has immobilized another nucleic acid which is also complementary to the target nucleic acid. Conversely, the biological sample can be introduced to a surface which has immobilized a complementary nucleic acid for immobilization of the target nucleic acid. The labeled probe can then be introduced to the system for binding to the immobilized target molecule. The excess labeled probe is then washed away and the resultant fluorescent intensity is correlated with fluorescence intensity from a standard curve to arrive at a concentration of the nucleic acid in the sample.

Those skilled in the art will recognize that many variations of immunoassays and nucleic acid assays can be performed and the inventive teachings in the instant invention for the use of novel dye systems can be used to develop novel adaptations to existing technologies.

Those skilled in the art will appreciate that the novel fluorescent particles and dyes described herein have many uses in immunoassays, fluorescence microscopy, in vivo imaging, in vitro cancer therapy, nucleic acid assays, and cell sorters.

### Experimental Section

Fluorescence measurements referred to in the following Examples were performed on a Perkin-Elmer model LS 50B Luminescence Spectrometer for dyes emitting up to around 780 nm. In some instances, as indicated in Table 1 by describing the Intensity in terms of nanoamps (nA), dyes emitting above 800 nm were measured according to Example 18. The fluorescence intensities are not corrected. Absorbance measurements were performed on a Hewlett Packard 8452A Diode Array Spectrophotometer.

### Preparation Example 1

### Synthesis of Silicon Phthalocyanine Dihydroxide SiPc(OH)₂

A suspension of silicon phthalocyanine dichloride (1.83 g, 3.0 mmol) in pyridine (50 ml) and water (50 ml) was refluxed with stirring on an oil bath at 120°C for 18 hours. After cooling the dark blue solid product was filtered and the residue was washed with water (10 ml), acetone (5 ml) and then dried under vacuum to afford 1.71 g of the title compound.

### Preparation Example 2

### Synthesis of Silicon 2,3-Naphthalocyanine Dihydroxide (Hereinafter sometimes referred to as NaPcSi hydroxide)

A suspension of silicon 2,3-naphthalocyanine dichloride (280 mg, 0.34 mmol) in pyridine (10 ml) and water (10 ml) was refluxed with stirring on an oil bath at 130 °C for 24 hours. After cooling to room temperature, the dark green solid product was filtered and, the residue was washed, successively, with water (5 ml) and acetone (2 ml). The product was dried under vacuum to afford 217 mg of the title compound.

### Preparation Example 3

### Synthesis of 4,9-diethoxy-1,3-diiminobenz[f]isoindoline

Anhydrous ammonia was slowly bubbled through a stirred mixture of 1,4-diethoxynaphthalene-2,3-dicarbonitrile (1.33 g), 25% sodium methoxide in methanol (1.14 ml), and dry ethanol (10 ml) for 20 minutes. With continued ammonia introduction, the mixture was refluxed for 2 hours. After the resultant had cooled, the solvent was removed under vacuum with a rotary evaporator. The residue was treated with methylene chloride (10 ml) and the product was coliected by filtration, washed sequentially with water (5 ml) and methylene chloride (5 ml), vacuum dried and weighed (766 mg).

### Preparation Example 4

### Synthesis of [2¹,2⁶,12¹,12⁶-tetraphenyldinaphtho[b,1]-7,17 -dibenzo[g,q]-5,10,15,20-tetraazoporphyrinato]silicon dihydroxide (abbreviated as: Silicon [di(1,6-diphenyl-2,3-naphthalocyanine)] diphthalocyanine dihydroxide)

Silicon tetrachloride (231 *µ*L) was added to a mixture of diphenyl-1,3-diiminobenz[f]isoindoline (470 mg) and 1,3-diiminoisoindoline (97 mg) in freshly distilled quinoline (5 ml) under an argon atmosphere and the mixture heated with stirring at 200 °C for 40 minutes. The resultant was allowed to cool to 160 °C, treated with water (5 ml) and refluxed for 5 minutes. The mixture was cooled, treated with ether (30 ml) and filtered washing the solid sequentially with ether (10 ml) and water (10 ml). The organic layer of the filtrate (which was dark green) was separated from the aqueous layer, washed with water (15 ml), dried (MgSO₄) and evaporated with a rotary evaporator. The residue was chromatographed three times on a silica gel (70-230 mesh, 60 Å,) column (2x50 cm) equilibrated in hexane and eluted sequentially with hexane, hexane - 10 % methylene chloride, hexane - 20% methylene chloride, and finally hexane - 50% methylene chloride. The product was vacuum dried and weighed (55.5 mg).

UV-vis (tetrahydrofuran) (λₘₐₓ(nm), ∈ (M⁻¹ cm⁻¹) ) : 640; 680; 714, 67900; 742.

Fluorescence (tetrahydrofuran) (λₘₐₓ(nm) ) : 750.

### Preparation Example 5

### Synthesis of 5,6-dichloro-1,3-diiminoisoindoline

Anhydrous ammonia was slowly bubbled through a stirred mixture of 4,5-dichlorphthalonitrile (1.0 g), 8% sodium butoxide in 1-butanol (500 *µ*L), 1,4-dioxane (1 ml), and dry 1-butanol (10 ml) for 60 minutes. With continued ammonia introduction, the mixture was refluxed for 2 hours. After the resultant had cooled, the product was collected by filtration, washed with methylene chloride (20 ml), vacuum dried and weighed (0.63 g).

### Example 1

### Synthesis of [2¹,2⁶,12¹,12⁶-tetraphenyldinaphtho[b,l]-7.17-dibenzo(g.q]-5,10,15,20-tetraazoporphyrinato]silicon bis [poly(ethylene glycol)methyl ether] (abbreviated as: Silicon [di(1,6-diphenyl-2,3 naphthalocyanine)]diphthalocyanine bis [poly(ethylene glycol) methyl ether])

A mixture of Silicon[di(1,6-diphenyl-2,3-naphthalocyanine)]diphthalocyanine dihydroxide (49 mg), poly(ethyleneglycol)methyl ether (400 mg), and 1,2,4-trimethylbenzene (5 ml) was refluxed with stirring on an oil bath at 220°C for 3 days using a Dean-Stark trap. The resultant was concentrated under vacuum on the rotary evaporator. The residue was chromatographed on a silica gel (70-230 mesh, 60 Å)column(2x50 cm) equilibrated in methylene chloride and eluted sequentially with methylene chloride - 1% isopropanol, methylene chloride - 5% isopropanol, methylene chloride - 20% isopropanol, methylene chloride - 50% isopropanol and finally methylene chloride - 50% methanol. The product was vacuum dried and weighed (145 mg).

UV-vis (tetrahydrofuran) (λₘₐₓ(nm) ) : 648, 692, 726,758

Fluorescence (tetrahydrofuran) (λₘₐₓ(nm) ) : 765

### Example 2

### Synthesis of [2¹,2⁶,12¹,12⁶-tetraphenyldinaphtho[b,l]-7.17-dibenzo(g,q]-5,10,15,20-tetraazoporphyrinato]silicon bis [(4-octyl)phenoxide] (abbreviated as: Silicon[di(1,6-diphenyl-2,3 naphthalocyanine)]diphthalocyanine bis [(4-octyl)phenoxide])

A mixture of Silicon [di(1,6-diphenyl-2,3-naphthalocyanine)]diphthalocyanine dihydroxide (42 mg), 4-octylphenol (41 mg) and 1,2,4 - trimethylbenzene (5 ml) was refluxed with stirring on an oil bath at 200°C for 16 hours. The resultant was concentrated under vacuum on the rotary evaporator. The residue was chromatographed on a silica gel (70-230 mesh, 60 Å)column (2x50 cm) equilibrated in hexane and eluted with hexane - 50% methylene chloride. The product was vacuum dried and weighed (49 mg).

UV-vis (tetrahydrofuran) (λₘₐₓ(nm) ) : 644, 684, 716, 746

Fluorescence (tetrahydrofuran) (λₘₐₓ(nm) ) : 751

### Example 3

### Silicon 2, 3-naphthalocyanine bis(dimethyloctadecylsilyloxide)

A mixture of Silicon 2,3-naphthalocyanine dihydroxide (155 mg), chlorodimethyloctadecylsilane (1.04g), imidazole (204 mg) and dimethylformamide (5 *µ* L) was stirred at room temperature for 1 hour. The resultant was concentrated under vacuum on the rotary evaporator. The residue was chromatographed on a silica gel (70-230 mesh, 60 Å)column(2x50 cm) equilibrated in hexane. The product was eluted sequentially with hexane and methylene chloride, vacuum dried and weighed (180 mg).

UV-vis (tetrahydrofuran) (λₘₐₓ(nm)):686, 732, 770

Fluorescence (tetrahydrofuran) (λₘₐₓ(nm)): 776

### Example 4

### Synthesis of [2¹,2⁶,12¹,12⁶-tetraphenyldinaphtho[b,l]-7,17-dibenzo(g,q]-5,10,15,20-tetraazoporphyrinato]silicon bis[poly(ethylene glycol) (abbreviated as: Silicon di[(1,6 diphenyl)-2.3-naphthalocyanineldiphthalocyanine bis [poly(ethylene glycol)])

A mixture of Silicon di[(1,6-diphenyl)-2,3 naphthalocyanine]diphthalocyanine dihydroxide (49 mg), poly(ethylene glycol) (1 g), and 1,2,4-trimethylbenzene (5 ml) was refluxed with stirring on an oil bath at 210°C for 3 days using a Dean-Stark trap. The resultant was concentrated under vacuum on the rotary evaporator. The residue was chromatographed on a silica gel(70-230 mesh, 60 Å)column (2x50 cm) equilibrated in methylene chloride and eluted sequentially with methylene chloride - 1% isopropanol, methylene chloride - 5% isopropanol, methylene chloride - 20% isopropanol and finally methylene chloride - 50% isopropanol. The product was then re-chromatographed on silica gel GF, 1000 *µ*, 20x20cm) plates eluting sequentially (air drying the plates between each elution) with methylene chloride, methylene chloride- 10% methanol and finally tetrahydrofuran. The product was vacuum dried and weighed (152 mg). NMR (500 MHZ, CDCl₃) δ8.30(m,4H), 8.25 (m,4H), 8.00(m,24H,) 7.77(m,4H), 3.63(m,CH₂'s)

UV-vis (tetrahydrofuran) (λₘₐₓ(nm) ) : 648, 692, 720, 754

Fluorescence (tetrahydrofuran) (λₘₐₓ(nm) ) : 760

### Example 5

### Synthesis of [2¹,2⁶,12¹,12⁶-tetraphenyldinaphtho[b,l]7,17-dibenzo[g,q],-5,10,15,20-tetraazoporphyrinato]silicon [poly(ethylene glycol)] [acetylthiopropionyl poly(ethylene glycol)](abbreviated as: Silicon di[(1,6-diphenyl)-2,3-naphthalocyanine] diphthalocyanine [poly(ethylene glycol)] [poly(ethylene glycol)acetylthiopropionate])

A mixture of acetylthiopropionic acid, (15 mg), 1,1'- carbonyldiimidazole (16 mg) and dimethylformamide (1 ml) was stirred at room temperature for 40 minutes. A portion of this solution ( 100 *µ*L) was added to Silicon di[(1,6-diphenyl)-2,3-naphthalocyanine]diphthalocyanine bis [poly(ethylene glycol)] (49.5 mg) and the mixture stirred at room temperature for 3 days. The resultant was concentrated under vacuum on the rotary evaporator. The residue was chromatographed on a silica gel (GF, 1000 *µ*, 20x20 cm) plate eluting with tetrahydrofuran, vacuum dried'and weighed (3 mg).

UV-vis (tetrahydrofuran) (λₘₐₓ(nm)) : 644, 690, 718, 750

Fluorescence (tetrahydrofuran) (λₘₐₓ(nm) ) : 754

### Example 6

### Synthesis of [2¹,2⁶,12¹,12⁶-tetraphenyldinaphtho[b,l]-7²,7³,17²,17³-tetracarboxydibenzo[g,q]-5,10,15,20-tetraazoporphyrinato]silicon dihydroxide (abbreviated as: Silicon di[(1,6-diphenyl)2,3-naphthalocyanine]di(2,3-dicarboxyphthalocyanine)dihydroxide)

A mixture of Silicon di[(1,6-diphenyl)-2,3-naphthalocyanine]di(2,3-dicyanophthalocyanine)dihydroxide (36 mg) and concentrated sulfuric acid (200 *µ*L) was heated with stirring at 50 °C for 48 hours. The cooled mixture was then carefully treated with water (150*µ* L) and heated with stirring at 100°C for 20 hours. The cooled mixture was then treated with water (1 ml) and the dark precipitate collected by filtration washing with water (1 ml). The solid was then treated with 1 N potassium carbonate solution (1 ml) and refluxed with stirring for 1 hour. The cooled mixture was acidified to pH 2 by dropwise addition of 6 N hydrochloric acid and the fine dark green solid product filtered, washing with water (1 ml). The solid was vacuum dried and weighed (20 mg).

UV-vis (tetrahydrofuran) (λₘₐₓ(nm) ) : 636, 658, 716, 788.

Fluorescence (tetrahydrofuran) (λₘₐₓ(nm)) : 791.

### Example 7

### Synthesis of [2¹,2⁶,12¹,12⁶-tetraphenyldinaphtho[b,l]-7²,7³,17²,17³-tetracarboxydibenzo[g,q]-5,10,15,20-tetraazoporphyrinato]silicon bis [poly(ethylene glycol)methyl ether](abbreviated as: Silicon di[(1,6-diphenyl) 2,3-naphthalocyanine]di(2,3-dicarboxyphthalocyanine) bis[poly(ethylene glycol)methyl ether])

A mixture of Silicon di[(1,6-diphenyl)-2,3-naphthalocyanine]di(2,3-dicarboxyphthalocyanine) dihydroxide (10 mg), poly(ethylene glycol)methyl ether (80 mg) and 1,2,4-trimethylbenzene (1 ml) was refluxed with stirring on an oil bath at 220°C for 3 days using a Dean-Stark trap. The resultant was concentrated under vacuum on the rotary evaporator. The residue was chromatographed on a silica gel (GF, 1000 *µ*, 20x20 cm) plate eluting with methylene chloride - 10% methanol, air drying the plate and re-eluting with methylene chloride - 10 % methanol. The green product was vacuum dried and weighed (8 mg).

IR (KBr)1712 cm⁻¹ (COOH)

UV-vis (tetrahydrofuran) (λₘₐₓ(nm) ) : 648,702,726,792.

UV-vis (water) (λₘₐₓ(nm) ) : 712, 816.

Fluorescence (tetrahydrofuran) (λₘₐₓ(nm)): 800.

### Example 8

### Synthesis of Silicon (IV)2,3-naphthalocyanine bis(tert -butyldimethylsilyloxide)

A mixture of silicon naphthalocyanine dihydroxide, tert-butyldimethylchlorosilane (390 mg), imidazole (180 mg) and dimethylformamide (5 ml) was stirred at 150°C for 30 minutes. The resultant was chromatographed on a silica gel (70-230 mesh, 60 Å)column (2x50 cm) equilibrated in hexane. The product was eluted sequentially with hexane and toluene, vacuum dried and weighed (6 mg).

UV-vis (tetrahydrofuran) (λₘₐₓ(nm) ) : 772,730, 686.

Fluorescence (tetrahydrofuran) (λₘₐₓ(nm) ) : 775.

¹H-NMR (500 MHZ, CDCl₃) δ 10.14 (s,8H), 8.67(m,8H), 7.90(m,8H), -1.20(s,18H), -2.60(s,12H)

### Example 9

### Synthesis of Silicon (IV) phthalocyanine bis(tert-butyl-dimethylsilyloxide)

A mixture of Silicon(IV) phthalocyanine dihydroxide (200 mg), tert-butyldimethylchlorosilane (525 mg), imidazole (272 mg) and dimethylformamide (5 ml) was stirred at 150°C for 30 minutes. The resultant was chromatographed on a silica gel (70-230 mesh, 60 Å)column (2x50 cm) equilibrated in hexane. The product was eluted sequentially with hexane and toluene, vacuum dried and weighed (12 mg).

UV-vis (tetrahydrofuran) (λₘₐₓ(nm) ) : 666,636,600.

Fluorescence (tetrahydrofuran) (λₘₐₓ(nm) ) : 671.

¹H-NMR (500 MHZ, CDCl₃) δ 9.65(m,8H), 8.33(m,8H), -1.45(s,18H),-2.98(s,12H)

### Example 10

### Synthesis of [2¹,2²-dichlorobenzo[b]-7,12,17-tri(2,3-naphtho)[g,l,q]-5,10,15,20,-tetraazoporphyrinato]silicon dihydroxide (abbreviated as: Silicon [tri(2,3-naphthalocyanine)]2,3-dichlorophthalocyanine dihydroxide)

Silicon tetrachloride (600 *µ*L) was added to a mixture of 5,6-dichloro 1,3-diiminoisoindoline (100 mg) and 1,3-diiminobenz[f]isoindoline(466 mg) in freshly distilled quinoline (4 ml) under an argon atmosphere and the mixture heated with stirring at 210°C for 2 hours. The resultant was allowed to cool, treated with water (20 ml) and refluxed for 20 minutes. The mixture was cooled, treated with ether (10 ml) and filtered, the solid was washed sequentially with water (2x20 ml), ether (3x20 ml), methylene chloride (10 ml) and acetone (20 ml). The solid was vacuum dried and weighed (0.83 g). The crude product was used without purification for the next step.

### Example 11

### Synthesis of [2¹,2²-dichlorobenzo[b]-7,12,17-tri(2,3-naphtho) [g,l,q]-5,10,15,20,-tetraazoporphyrinato] silicon bis(7-oct-1-enyldimethylsilyloxide) (abbreviated as: Silicon[tri(2,3-naphthalocyanine)] 2,3-dichlorophthalocyanine bis(dimethylhexylvinylsilyloxide))

A mixture of silicon[tri(2,3-naphthalocyanine)2,3-dichlorophthalocyanine dihydroxide (400 mg) and 7-oct-1-enyldimethylchlorosilane (1.5 ml) was stirred at room temperature for 15 hours. The resultant was concentrated under vacuum on the rotary evaporator. The residue was chromatographed on a silica gel (70-230 mesh, 60Å) column (2x50 cm) equilibrated in hexane. The product was eluted with toluene, vacuum dried and weighed (35 mg).

UV-vis (tetrahydrofuran) (λₘₐₓ(nm)∈(M⁻¹cm⁻¹)): 770, 728, 688, 654, 182000.

Fluorescence (tetrahydrofuran) (λₘₐₓ(nm)): 774, 727.

### Example 12 Synthesis of [2¹,2²-dichlorobenzo[b]-7,12,17-tri(2,3-naphtho) [g,l,q] -5,10,15,20,-tetraazoporphyrinatol silicon bis[dimethylpentafluorophenylsilyloxide)] (abbreviated as: Silicon[tri (2,3-naphthalocyanine)]2,3-dichlorophthalocyanine bis (dimethylpentafluorophenylsilyloxide))

A mixture of silicon[tri(2,3-naphthalocyanine)]2,3-dichlorophthalocyanine dihydroxide (400 mg), chlorodimethylpentafluorophenylsilane (1.0 ml), imidazole 270 mg) and dimethylformamide (5 ml) was stirred at room temperature for 16 hours. The reaction mixture was filtered, washing the solid with dimethylformamide(4x2 ml). The filtrate was evaporated under vacuum on the rotary evaporator. The residue was dissolved in toluene and filtered. The filtrate was concentrated under vacuum on the rotary evaporator. The residue was chromatographed on a silica gel (70-230 mesh, 60Å) column (2x50 cm) equilibrated in hexane. The product was eluted sequentially with hexane and toluene, vacuum dried and weighed (34 mg).

UV-vis (tetrahydrofuran) (λₘₐₓ (nm) ∈ (M⁻¹cm⁻¹) ) : 780, 736, 696, 662; 142000.

Fluorescence (tetrahydrofuran) (λₘₐₓ (nm)): 735, 784 nm.

### Example 13

### Synthesis of [2¹,2⁶,12¹,12⁶-tetraphenyldinaphtho[b,l]-7,17-di(2,3-naphtho) [g,q]-5,10,15,20-tetraazoporphyrinatolsilicon bis (7-oct-1-enyldimethylsilyloxide) (abbreviated as: Silicon[di(1,6-diphenylnaphthalocyanine)]dinaphthalocyanine bis(dimethylhexylvinylsilyloxide))

A mixture of silicon[di(1,6-diphenylnaphthalocyanine)]di-2,3-naphthalocyanine dihydroxide (25 mg) and 7-oct-1-enyldimethychlorosilane(60 *µ*L), imidazole (16 mg) and dimethylformamide (4 ml) was stirred at room temperature for 3 days. The resultant was concentrated under vacuum on the rotary evaporator. The residue was chromatographed on a silica gel (70-230 mesh, 60Å) column (2x50 cm) equilibrated in hexane. The product was eluted sequentially with hexane and toluene, vacuum dried and weighed (15 mg). This compound has also been isolated as a by-product during the chromatographic purification in Example 75.

UV-vis (tetrahydrofuran) (λₘₐₓ(nm)∈(M⁻¹cm⁻¹) : 786, 440000.

Fluorescence (tetrahydrofuran) (λₘₐₓ(nm)): 792.

¹H-NMR (500 MHZ, CDCl₃) : δ -2.9(S,1211) ;-2.0(m,4H), 1.07(m,4H), -0.06(m,4H),0.17(m,4H), 0.6(m,4H), 1.4(m,4H), 4.7(m,4H), 5.3(m,2H), 7.8(m,8H), 8.03(m,16H) ,8.15(m,4H), 8.38(m,8H), 8.8 (m,4H).

### Example 14

### Synthesis of [2¹,2⁶,12¹,12⁶-tetraphenyldinaphtho[b,l]-7,17-di(2,3-naphtho) [g,q]-5,10,15,20-tetraazoporphyrinato]silicon dihydroxide (abbreviated as:Silicon[di(1,6-diphenylnapththalocyanine)]dinaphthalo cyanine dihydroxide))

Silicon tetrachloride (600 *µ*L) was added to a mixture of 4,9-diphenyl 1,3-diiminobenz[f]isoindoline (1.0 g) and 1,3 diiminobenz[f]isoindoline (50 mg) in freshly distilled quinoline (7 ml) under an argon atmosphere and the mixture heated with stirring at 210°C for 2 hours. The resultant was allowed to cool, treated with water (10 ml) and refluxed for 15 minutes. The mixture was cooled, treated with ether (20 ml) and filtered. The organic layer of the filtrate was washed with 1 N hydrochloric acid (2x20ml). The solid was washed with methylene chloride (5x20ml). The organic phases were combined and evaporated with a rotary evaporator. The residue was chromatographed on a silica gel (70-230 mesh, 60Å) column (2x50 cm) equilibrated in methylene chloride. The product was eluted with toluene - 10% isopropanol, vacuum dried and weighed (25 mg).

UV-vis (methylene chloride) (λₘₐₓ(nm) : 794.

### Example 15

### Synthesis of Silicon (IV) phthalocyanine bis(7-oct-1-enyl dimethylsilyloxide (abbreviated as: Silicon phthalocyanine bis(dimethylhexylvinyloxide))

A mixture of silicon phthalocyanine dihydroxide 500 mg), 7-oct-1-enyldimethylchlorosilane (2.5 ml), imidazole (680 mg)and dimethylformamide(10 ml) was stirred at room temperature for 48 hours. The resultant was evaporated under vacuum on the rotary evaporator. The residue was dissolved in toluene (20 ml) and filtered. The solid washed with toluene(40 ml). The filtrate was concentrated under vacuum on the rotary evaporator and was chromatographed on a silica gel (70-230 mesh, 60Å)column (2x50 cm) equilibrated in hexane. The product was eluted sequentially with hexane and toluene, vacuum dried and weighed (324 mg).

UV-vis (tetrahydrofuran) (λₘₐₓ(nm)∈(M⁻¹cm⁻¹): 668, 636,660, 283000

Fluorescence (tetrahydrofuran) (λₘₐₓ(nm) ) : 673

¹H-NMR(500 MHZ, CDCl₃) : δ -2.8(s,12H), -2.27 (m,4H), -1.33 (m,4H), -0.20(m,4H), 0.31 (m,4H), 0.84(m,4H), 1.54(m,4H), 1.80 (m,4H), 4.94(m,4H), 5.75(m,2H), 8.3(m,8H),9.65(m,8H).

### Example 16

### Synthesis of Silicon(IV) phthalocyanine(10-carbomethoxy decyldimethylsilyloxide) (dimethylvinylsilyloxide)

A mixture of silicon(IV) phthalocyanine dihydroxide (500 mg), imidazole (300 mg), dimethylformamide (6 ml) and a mixture of (10-carbomethoxydecyldimethylchlorosilane (590 mg) and chlorodimethylvinylsilane (250 mg) was added and the reaction mixture stirred at room temperature for 24 hours. The resultant was concentrated under vacuum on the rotary evaporator. The residue was chromatographed on a silica gel (70-230 mesh, 60Å)column (2x50 cm) equilibrated in hexane. The products (a) Silicon (IV) phthalocyanine bis(10-carbomethoxy decyldimethyl silyloxide) (100 mg) and (b) silicon(IV) phthalocyanine (10-carbomethoxydecyl-dimethylsilyloxide)(dimethylvinylsilyloxide)(68 mg) were eluted with toluene.
(a) UV-vis (tetrahydrofuran) (λₘₐₓ(nm) : 666,638,602. Fluorescence (tetrahydrofuran) (λₘₐₓ(nm) ) : 671. ¹H-NMR(500 MHZ, CDCl₃) : δ -2.90(s,12H) ,2.27(m,4H), -1.35(m,4H), -.22(m,4H), 0.25(m,4H), 1,18 (m, 4H), 1.0(m,4H), 0.70 m,4H)1.65(m,4H), 2.35(m,4H), 3.7(s,6H) 8.33(m,8H), 9.64(m,8H).
(b) UV-vis (tetrahydrofuran) (λₘₐₓ(nm) ) : 668, 636,602. Fluorescence (tetrahydrofuran) (λₘₐₓ(nm) ) : 673. ¹H-NMR(500 MHZ, CDCl₃) : δ -2.9(s,6H), -2.75(s,6H), -2.27(m,4H), -1.36(m,4H), -0.015(m,4H), 0.027 (m,4H), 0.07 m,4H),0.10(m,4H), 1.21(m,4H), 1.65(m,3H), 2.33(m,3H);3.0(m,1H), 3.4(m,1H), 3.6(s), 3.7(s), 4.26(m,1H);8.33(m,8H), 9.6 (m, 8H).

### Example 17

### Synthesis of Sulfo[2¹,2⁶,12¹,12⁶-tetraphenyldinaphtho[b,l]-7,17-dibenzo[g,q]-5,10, 15.20-tetraazoporphyrinatolsilicon dihydroxide (abbreviated as: Sulfo Silicon di[(1,6-diphenyl)-2.3-naphthalocyanine] diphthalocyanine dihydroxide)

A mixture of Silicon di[(1,6-diphenyl)-2,3-naphthalocyanine]diphthalocyanine)dihydroxide (0.2 g) and chloroform (2 ml) was stirred at room temperature for 10 minutes under an argon atmosphere. The mixture was then cooled in an ice-bath and chlorosulfonic acid (2 ml) was added. The mixture was stirred in the ice-bath for 15 minutes and then at room temperature for 20 minutes. The mixture was then refluxed for 2 hours, cooled and poured onto crushed ice (100 g). The resulting green mixture was extracted with chloroform (2x30 ml). The combined organic layers were washed with water (20 ml), dried (MgSO₄) and evaporated with a rotary evaporator. The brown residue was treated with 6 N potassium hydroxide (3 ml) with swirling and after 5 minutes the mixture was partitioned between water (40 ml) and ether (20 ml). The aqueous layer was acidified with 1 N hydrochloric acid (15 ml), washed with ether (40 ml) and evaporated with a rotary evaporator. The residue was vacuum dried and weighed (8 mg).

UV-vis(methanol) ( (λₘₐₓ(nm) ) : 650, 658, 692, 726, 748(sh).

UV-vis (water) ((λₘₐₓ(nm)): 654, 662, 732, 758 (sh). Fluorescence (water) ( (λₘₐₓ (nm) ) : 773.

IR(KBr) (cm¹) : 3153, 1720, 1405, 1225, 1182, 1037, 1014, 622.

### Example 18

### Synthesis of Acetylthiopropionic Acid

To a stirred solution of 3-mercaptopropionic acid (7 ml), and imidazole (5.4 g) in tetrahydrofuran (700 ml) was added, dropwise, over 15 minutes, under argon, a solution of 1-acetylimidazole (9.6 g) in tetrahydrofuran (100 ml). The solution was allowed to stir a further 3 hours at room temperature after which time the tetrahydrofuran was removed under vacuum. The residue was treated with ice-cold water (18 ml) and the resulting solution acidified with ice-cold concentrated hydrochloric acid (14.5 ml) to pH 1.5-2.0 The mixture was extracted with diethyl ether (2x50 ml), the ether was washed with water (2x50 ml) and dried over MgSO₄ and evaporated. The residual crude yellow oily solid product (10.5 g) was recrystallized from chloroform-hexane to afford 4.8 g (41% yield) acetylthiopropionic acid as a white solid with a melting point of 44° - 45°C.

### Example 19

### Synthesis of [2¹,2⁶,12¹,12⁶-tetraphenyldinaphtho[b,l]7,17-dibenzo[g,q],-5,10,15,20-tetraazoporphyrinato]silicon [poly(ethylene glycol)] [thiopropionyl poly(ethylene glycol)] (abbreviated as: Silicon di[(1,6-diphenyl)-2,3-naphthalocyanine] diphthalocyanine [poly(ethylene glycol)] [poly(ethylene glycol)thiopropionate])

A solution of silicon di[(1,6-diphenyl)-2,3-naphthalocyanine]diphthalocyanine[poly(ethyleneglycol thiopropionate] in 0.12M potassium carbonate in 80% methanol (1 ml) was allowed to stand at room temperature for 5 minutes. The pH of the solution was then adjusted to 7 by dropwise addition of a solution of 0.5 M potassium phosphate pH 7 which was made 1N in hydrochloric acid. The thiol content of the solution was estimated by Ellman's method using dithionitrobenzoic acid. The title compound in solution is capable of being conjugated to ligand analogues, proteins, polypeptides and nucleic acids containing for example, maleimide or alkyliodide functional groups.

### Example 20

### Synthesis of 2(2-amino-4-thiolbutanoic acid thiolactone)-bromoacetamide(abbreviated as: bromoacetyl-HCTL)

Bromoacetic acid (1.0 g), homocysteine thiolactone hydrochloride (1.1 g) and pyridine (1.2 ml) were dissolved in anhydrous dimethylformamide (36 ml) and 1-(3-dimethylaminopropyl)-3-ethylcarbodiimide hydrochloride (1.52 g) was added. The reaction was stirred at room temperature for 18 hours. The solvents were removed under vacuum, ethanol (10 ml) was added to dissolve the residue and then the ethanol was removed under vacuum. Ethanol (10 ml) was again added to dissolve the residue and was again removed under vacuum. Water (20 ml) was added to the oil and the aqueous solution was extracted 3 times with methylene chloride (45 ml). The combined organic extracts were dried over anhydrous magnesium sulfate. The solution was filtered and the solvent was removed under vacuum to give a clear oil. Diethyl ether (5 ml) was added and the resulting precipitate was collected and washed on a fritted funnel. The precipitate was dried under vacuum and 1.0 g of the title compound was recovered.

### Example 21

### Synthesis of [2-naphtho[b]-7,12,17-tribenzo[g,l,q]-5,10,15,20-tetraazoporphyrinato]silicon dihydroxide (abbreviated as: Silicon (IV) [tri(phthalo)naphthalocyanine]hydroxide

Silicon tetrachloride (912 *µ*L) was added to a mixture of 1,3-diiminoisoindoline (1.0 g) and 1,3-diiminobenz[f]isoindoline (0.25 g) in freshly distilled quinoline (3 ml) under an argon atmosphere and the mixture heated with stirring at 210°C for 2 hours. The resultant was allowed to cool, treated with water (25 ml) and refluxed for 15 minutes. The mixture was cooled, the solid filtered, washing the solid sequentially with water (3x10 ml) and ether (5x10 ml). The solid was vacuum dried and weighed (1.5 g).

### Example 22

### Synthesis of [2-naphtho[b]-7,12,17-tribenzo[g,l,q]-5,10,15,20-tetraazoporphyrinato]silicon bis (7-oct-1-enyldimethyl silyloxide) (abbreviated as: Silicon [tri(phthalo)naphthalocyanine] bis(dimethylhexylvinylsilyloxide))

A mixture of Silicon (IV) [tri(phthalo)naphthalocyanine dihydroxide (1.0 g). 7-oct-1-enyldimethylchlorosilane (3.0 ml), imidazole (0.68 g) and dimethylformamide (10 ml) was stirred at room temperature for 24 hours. The resultant was concentrated under vacuum on the rotary evaporator. The residue was chromatographed on a silica gel (70-230 mesh 60A°) column (2x50 cm) equilibrated in hexane. The product was eluted sequentially with hexane and hexane - 3% toluene, vacuum dried and weighed (11 mg).

UV-vis(methylene chloride) ( (λₘₐₓ (nm) ) : 716, 704, 684, 648, 618

Fluorescence (methylene chloride) ( (λₘₐₓ (nm) ) : 710

¹H-NMR(500MHz,CDCl₃): δ -2.8(s,12H), -2.2(m,4H),-1.23(m,4H), -0.16(m,4H), 0.27 (m, 4h), 0.78 (m, 4H), 1.7(m,4H), 4.9(m,4H), 5.7(m,2H), 7.94(m,2H), 8.3(m,6H), 8.7(m,2H), 9.6(m,6H), 10.1(S,2H).

### Example 23

### Synthesis of Sulfo[2¹,2⁶,12¹,12⁶-tetraphenyldinaphtho[b,l]7,17-dibenzo[g,q]-5,10,15,20-tetraazoporphyrinato]silicon[N-(2-butyrothiolactone)amidomethoxide]hydroxide (abbreviated as Sulfo silicon di[(1,6-diphenyl)-2.3-naphthalocyanine]diphthalocyanine[-2-butyrothiolactone)amidomethoxide]hydroxide

A mixture of sulfo silicon di[(1,6-diphenyl)-2,3-naphthalocyanine]diphthalocyanine dihydroxide (200 mg), bromoacetyl homocysteine thiolactone (7 mg) and powdered potassium carbonate (180 mg), in dimethylformamide (2 ml) was stirred under argon at room temperature for 24 hours. The solvent was evaporated with a rotary evaporator, the residue treated with ethanol (2 ml) and filtered washing with ethanol (2 ml). The filtrate was evaporated, and the product vacuum dried and weighed ( 200 mg). This product was used without further purification in the next step.

### Example 24

### Synthesis of Sulfo[2¹,2⁶,12¹,12⁶-tetraphenyldinaphtho[b,l]7,17-dibenzo[g,q]-5,10,15,20-tetraazoporphyrinato]silicon[N-(cysteine)amidomethoxide]hydroxide (abbreviated as: Sulfo silicon di[(1,6-diphenyl)-2,3-naphthalocyanine]diphthalocyanine[N-(cysteine)amidomethoxide]hydroxide)

A solution of sulfo silicon di[(1,6-diphenyl-2,3-naphthalocyanine]diphthalocyanine[N-(2-butyrothiolactone)amidomethoxide]hydroxide(10mg) in water (182 ml) was treated with 1 N potassium hydroxide solution (46 ml) and allowed to stand at room temperature for 10 minutes. The pH of the solution was then adjusted to 7 by dropwise addition of a solution of 0.5 M potassium phosphate pH 7 which was made 1 N in hydrochloric acid. The thiol content of the solution was estimated by Ellman's method using dithionitrobenzoic acid. The title compound in solution is capable of being conjugated to ligand analogues, proteins, polypeptides and nucleic acids containing, for example, maleimide or alkyliodide functional groups.

### Example 25

### Synthesis of Silicon tetra-tert-butylphthalocyanine bis [(4-aminobutyl) dimethylsilyloxidel

To a stirred solution of silicon tetra-tert-butyl phthalocyanine dihydroxide (800 mg) in pyridine (140 ml) was added 4-aminobutyldimethylmethoxysilane (950 *µ*L). The solution was heated to reflux and pyridine allowed to distill off until 50 ml of distillate had been collected. The solution was allowed to cool and the residual pyridine removed under vacuum. The residue was chromatographed on a silica gel (70-230 mesh, 60Å, 3x50 cm) column equilibrated in methylene chloride. The product was eluted sequentially with methylene chloride, tetrahydrofuran and finally tetrahydrofuran - 2% triethylamine. The dark blue product was vacuum dried and weighed (355 mg).

UV-vis (tetrahydrofuran) (λ max(nm)): 606, 644, 672.

### Example 26

### Synthesis of Sulfo[2¹,2⁶,12¹,12⁶-tetraphenyldinaphtho[b,l]-7,17-dibenzo[g,q]-5,10,15,20-tetraazoporphyrinato]silicon dihydroxide (abbreviated as: Sulfo Silicon di[(1,6-diphenyl)-2.3-naphthalocyanine] diphthalocyanine dihydroxide)

Silicon di[(1,6-diphenyl)-2,3-naphthalocyanine]diphthalocyanine dihydroxide (110 mg) was dissolved in (1 ml) concentrated sulfuric acid, and 10 minutes later chlorosulfonic acid (150 ml) was added. The reaction mixture was then heated in an oil bath (100-130°C) for 2.5 hours. The reaction mixture was allowed to cool to room temperature and poured onto crushed ice (30 g). The pH of the green solution was adjusted with solid potassium carbonate to pH = 9.0. The solvent was evaporated with a rotary evaporator. The residue was dissolved in 200 mM potassium phosphate buffer (pH = 7.0) and applied to a C18-column (12 cm x 2.5 cm) that was equilibrated in 200 mM potassium phosphate buffer (pH = 7.0). The column was washed with 200 mM potassium phosphate buffer (pH = 7.0) (50 ml) water (300 ml), and the product was eluted with a mixture of water and methanol 2:1 (v/v). The solvent was evaporated with a rotary evaporator. The residue was vacuum dried and weighed (137 mg).

UV-vis (Water) (λ ₘₐₓ₍ₙₘ₎) 658, 698, 732, 756(sh).

UV-vis (Methanol) (λ ₘₐₓ₍ₙₘ₎) in neoH = 648, 688, 724, 742(sh). IR(KBr) (cm⁻¹) 3629, 3465, 3065, 2593, 1721, 1622, 1521, 1422, 1353, 1335, 1284, 1194, 1088, 1039, 1013, 941, 906, 821, 760, 651, 620.

¹H-NMR (500 MHZ, DMSO-d₆) δ = -2.4(s, OH), 8.1(m, Ar - H).

### Example 27

### Synthesis of Sulfo[2¹,2⁶,12¹,12⁶-tetraphenyldinaphtho[b,l]-7,17-dibenzo[g,q]-5,10, 15,20-tetraazoporphyrinato]silicon bis (4-Aminobutyldimethylsilane) (abbreviated as: Sulfo Silicon di[(1,6-diphenyl-2,3-naphthalocyanine) phthalocyaninel bis (4-Aminobutyldimethylsilane)

To a suspension of sulfo silicon di[(1,6-diphenyl-2-3-naphthalocyanine)phthalocyanine] dihydroxide (32 mg) in pyridine (20 ml) was added 4- aminobutyldimethylmethoxysilane (50 ml), and the reaction mixture was heated in an oil bath (140°C) for 3 hours. The reaction mixture was allowed to cool to room temperature, and DMF(5 ml) was added followed by 4-aminobutyl-dimethylmethoxy-silane (100 ml). The reaction mixture was then refluxed for 16 hours. After cooling the solvent was evaporated with a rotary evaporator. The residue was dissolved in methanol (2 ml) and applied on a C₁₈ column. The column was washed with (200 mM) potassium phosphate buffer pH = 7.0 (20 ml), water (200 ml), water/methanol = 3:1 (v/v) (40 ml), water/methanol = 2:1 (v/v) (40 ml). The product was eluted with 95% methanol, the solvent was evaporated with a rotary evaporator, and the product was dried under vacuum and weighed (32 mg).

UV-vis (Water) (λ ₘₐₓ₍ₙₘ₎) 658, 696(sh), 730.

UV-vis (Methanol) (λ ₘₐₓ₍ₙₘ₎) 648, 686, 722, 748(sh).

### Example 28

### Synthesis of Sulfo[2¹,2⁶,12¹,12⁶-tetraphenyldinaphtho[b,l]-7.17-dibenzo[g,q]-5,10, 15,20-tetraazoporphyrinato]silicon bis (3-amino-propyldiisopropylsilyloxide) (abbreviated as: Sulfo Silicon di[(1,6-diphenyl-2.3-naphthalocyanine) phthalocyanine] bis- (3-amino-propyldiisopropylsilyloxide)

A mixture of sulfo silicon di[(1,6-diphenyl)-2-3-naphthalocyanine]phthalocyanine dihydroxide (50 mg) 3-amino-propyldiisopropylmethoxysilane (190 microliters) in toluene (2ml) was refluxed for 16 hours. After cooling to room temperature the solvent was evaporated with a rotary evaporator. The green oily residue was applied to a C₁₈ column. The column was washed with (200 mM) phosphate buffer (pH = 7.0) (50 ml), water (200 ml), water/ methanol [(3:1;(v/v)(20 ml)], water/methanol 2:1 (v/v). The product was eluted with 95% methanol. The solvent was evaporated with a rotary evaporator, and the residue was vacuum dried and weighed (40.0 mg).

UV-vis (Methanol) (λ ₘₐₓ₍ₙₘ₎) : 648, 686, 724, 744(sh).

### Example 29

### Synthesis of Sulfo[2¹,2⁶,12¹,12⁶-tetraphenyldinaphtho[b,l]-7,17-dibenzo[g,q]-5,10, 15,20-tetraazoporphyrinato]silicon bis-[(10-carbomethoxydecyl) dimethylsilyloxide] (abbreviated as: Sulfo Silicon di[(1,6-diphenyl)-2,3-naphthalocyanine] diphthalocyanine bis-[(10-carbomethoxydecyl) dimethylsilyloxide]

A mixture of imidazole (33 mg) and (10-carbomethoxydecyl) dimethylchlorosilane in (1.0 ml) pyridine was stirred for 1 hour at room temperature, and sulfo silicon di[(1,6-diphenyl)-2-3-naphthalocyanine]diphthalocyanine dihydroxide (20 mg) in pyridine (3 ml) was added. After stirring the reaction mixture for 16 hours, the pyridine was evaporated with a rotary evaporator. The residue was triturated with (2 ml) (200 mM) potassium phosphate buffer, PH = 7.0 (2 ml) (200mM) and applied to C₁₈ column (equilibrated with (200 mM) potassium phosphate buffer pH = 7.0). The column was washed with potassium phosphate buffer (60 ml) (200mM) (pH 7.0), water (210 ml), water/MeoH [(1:1; (v/v) (40 ml)], and water/MeOH [(1:2; (v/v) (35 ml)]. The product was then eluted with 95% methanol, the solvent was evaporated with a rotary evaporator. The residue was vacuum dried and weighed (8 mg)

UV-vis (Water) (λ ₘₐₓ₍ₙₘ₎) 658, 694, 730, 750,(sh).

UV-vis (Methanol) (λ ₘₐₓ₍ₙₘ₎) 650, 690, 726, 746(sh).

IR(KBr) (cm⁻¹) 2924,2854,1744.

Fluorescence (methanol) λ max (nm) : 752

Fluorescence (water) λ max (nm): 761

### Example 30

### Synthesis of Sulfo[2¹,2⁶,12¹,12⁶-tetraphenyldinaphtho[b,l]-7,17-dibenzo[g,q]-5,10, 15,20-tetraazoporphyrinato]silicon bis (7-oct-1-enyldimethylsilyloxide) (abbreviated as: Sulfo Silicon di [(1,6-diphenyl)-2.3-naphthalocyaninel diphthalocyanine bis (7-oct-1-enyldimethylsilyloxide)

A mixture of sulfo silicon di[(1,6-diphenyl)-2-3-naphthalocyanine]diphthalocyanine dihydroxide (10 ml) and imidazole (41 mg) in dimethylformamide (2 ml) was stirred at room temperature for 10 minutes and 7-oct-1-enyldimethylchlorosilane was added. The mixture was stirred for 14 hours at room temperature and the solvent was removed with a rotary evaporator. The residue was triturated with (2 ml) (200 mM) potassium phosphate buffer pH = 7.0 and applied to a C₁₈ column (equilibrated with 200 mM potassium phosphate buffer, pH = 7.0). The column was washed with potassium phosphate buffer (40 ml), water (150 ml) and water/ methanol (2:1 (v/v)). The product was eluted with 95% methanol, and the solvent was evaporated with a rotary evaporator. The residue was vacuum dried and weighed (9 mg).

¹H-NMR (500 MHZ, DMSO) δ -2.8(s,12H), -2.1(m,4H),-1.3(m,4H),

-0.23(m,4H), 0.06(m,4H), 0.5(m,4H), 1.3(m,4H), 4.7(m,4H), 5.4(m,2H), 8.0(Ar-H).

### Example 31

### Synthesis of Sulfo silicon naphthalocyanine bis(4-aminobutyldimethyl silyloxide

A mixture of sulfo silicon naphthalocyanine dihydroxide triethyl ammonium salt (30 mg) and pyridine was stirred at room temperature for 10 minutes, and then N,N-Diisopropylethylamine (10 ml) followed by 4-aminobutyldimethylmethoxysilane (380 microliters) were added. The reaction mixture was heated in an oil bath for 2 hours at 130°C. After cooling to room temperature the solvent was removed with a rotary evaporator and the residue was triturated with 200 mM potassium phosphate buffer pH = 7.0 (2 ml) and applied to a C₁₈ column (1.5x23 cm filled with C₁₈ to 7.0 cm height). The column was washed with 200 mM potassium phosphate buffer (40 ml), water (80 ml), water/methanol (2:1) (40 ml), water/methanol (2:1) (70 ml), and the major green fraction was eluted with water/methanol (1:3) (40 ml). The solvent was removed with a rotary evaporator and the residue was vacuum dried and weighed (14 mg). IR(KBr) (cm⁻¹) 3069, 2964, 1631, 1528, 1362, 1252, 1184, 1091, 1067, 1035, 844, 798, 761, 728, 691, 615.

¹H-NMR (500 MHZ, DMSO) δ -2.5(S,12H), -1.9(m,4H), -1.0(m,4H), 0.4(m,4H), 2.0(m,4H).

### Example 32

### Synthesis of Sulfo silicon naphthalocyanine bis [10-(carbomethoxy)decyldimethylsiloxide]

To a stirred solution of imidazole (109 mg) in pyridine (2 ml) was added 10-(carbomethoxy)decyldimethylchlorosilane (513 microliters), and the mixture stirred for 20 min. at room temperature. Sulfo silicon naphthalocyanine dihydroxide (60 mg) (neat) was then added followed by pyridine (1 ml) and 10-(carbomethoxy)decyldimethylchlorosilane (0.6 ml). The reaction mixture was allowed to stir 14 hours, and the solvent evaporated with a rotary evaporator. The residue was suspended in 40 mM potassium phosphate buffer (pH 7.0) (2 ml) and chromatographed on a C₁₈ column. After washing the column with 200 mM potassium phosphate buffer (40 ml) and water (300 ml), the product was eluted with water/methanol (1:1). The solvent was evaporated with a rotary evaporator. The residue was vacuum dried and weighed (55 mg).

### Example 33

### Synthesis of sulfo silicon naphthalocyanine bis(3-aminopropyldiisopropylsilane)

A mixture of sulfo silicon napthalocyanine (50 mg) and 3-aminopropyldiisopropylethoxysilane (200 ml) in toluene (3 ml) is refluxed for 16 hours. The reaction mixture is allowed to cool to room temperature and the solvent is evaporated with a rotary evaporator. The residue can be purified on a C₁₈ column, with (200 mM) potassium phosphate buffer, (pH = 7.0) water and 95% methanol.

### Example 34

### Synthesis of 1,4-diphenylnaphthalene -2,3-di-carbonitrile

In a dry 2L 3-necked round bottom flask equipped with a magnetic stirring bar, dropping funnel, gas inlet tube attached to an argon gas cylinder, was placed tetrahydro-1,4-diphenyl-1,4-epoxy-napthalene-2,3-dicarbonitrile (20 g) and dry tetrahydrofuran (450 ml) while purging the flask with argon gas. The mixture was stirred for 20 minutes. The flask was cooled to -78°C (acetone/dry ice) and Lithium bis(trimethylsilyl)- amide (150 ml, 1.0 M THF) was added dropwise over 2 hours. The mixture was allowed to stir at this temperature, and saturated ammonium chloride (300 ml) was added. The mixture was allowed to warm to room temperature, and the white solid was filtered off. The organic layer of the filtrate was separated. The aqueous layer was washed with ether (100 ml). The combined organic layers were dried (magnesium sulfate). After the magnesium sulfate was filtered off, the solvent was evaporated with a rotary evaporator, the residue triturated with ether, and the solid filtered, dried under vacuum and weighed (17 g.)

IR(KBr) (cm⁻¹) 3059, 2232, 1608, 1494, 1446, 1400, 1378, 1183, 1077, 1029, 1001, 931, 796, 783, 757, 706, 681, 657, 620, 517, 437.

¹H-NMR (500 MHZ, DMSO) δ 7.5(m,4H), 7.6(m,8H), 7.8(m,2H).

### Example 35

### Synthesis of Sulfo[2¹,2⁶,12¹,12⁶-tetraphenyldinaphtho[b,l]-7.17-dibenzo[g,q]-5,10, 15,20-tetraazoporphyrinato]silicon bis [N-succinamido)aminobutyldimethyl silyloxide

A mixture of sulfo silicon di[(1,6-diphenyl)-2,3-napthalocyanine]diphthalocyanine bis (4-aminobutyldimethylsilyloxide)(20mg) and succinic anhydride (50 mg) in dimethylformamide (4 ml) is refluxed for 2 hours. The reaction mixture is allowed to cool to room temperature and the solvent is evaporated with a rotary evaporator. The residue can be purified on a C₁₈ column, with (200mM) potassium phosphate buffer, (pH 7.0), water and methanol.

### Example 36

### Synthesis of Sulfo[2¹,2⁶,12¹,12⁶-tetraphenyldinaphtho[b,l]-7.17-dibenzo[g,q]-5,10,15,20-tetraazoporphyrinato]silicon bis[4[(acetylthiopropionamido)butyl] dimethylsilyloxide] (Abbreviated as: Sulfo Silicon di[(1,6-diphenyl)-2.3-naphthalocyanine] diphthalocyanine bis ((acetylthiopropionamido)butyl silyloxide)

A mixture of sulfo silicon di[(1,6-diphenyl)-2,3-napthalocyanine]diphthalocyanine bis (4-aminobutyldimethylsilyloxide) in dimethylformamide and a solution of acetylthioproponic acid and 1,1'-Carbonyldiimidazole in dimethylformamide is stirred at room temperature for 1 hour. The solvent is evaporated with a rotary evaporator. The residue can be purified on a C₁₈ column, with (200 mM) potassium phosphate buffer (pH 7.0) water and methanol.

### Example 37

### Synthesis of Sulfo[2¹,2⁶,12¹,12⁶-tetraphenyldinaphtho[b,l]-7.17-dibenzo[g,q]-5,10, 15,20-tetraazoporphyrinato]silicon bis[4[(thiopropionamido)butyl] dimethylsilyloxide] (Abbreviated as: Sulfo Silicon di[(1,6-diphenyl) -2.3-naphthalocyanine] diphthalocyanine bis ((thiopropionamido)butyl dimethyl silyloxide)

A mixture of sulfo silicon di[(1,6-diphenyl-2,3-naphthalocyanine] diphthalocyanine bis ((acetylthiopropionamido)butyl dimethyl silyloxide) in 50% (v/v) aqueous methanol (20 mM) and potassium carbonate at 200 mM is stirred at room temperature for 20 min. The mixture is neutralized to pH 7 with 1 N hydrochloric acid and the solvent is evaporated with a rotary evaporator. The residue can be purified on a C₁₈ column, with (200mM) potassium phosphate buffer (pH 7.0), water and methanol.

### Example 38

### Preparation of a Conjugate of Sulfonated Hybrid Phthalocyanine Derivative and an Antibody

A monoclonal antibody against human chorionic gonadotropin (Calbiochem, San Diego, CA) at 10 mg/ml in 50 mM potassium phosphate, 150 mM sodium chloride, pH 7.0, is reacted with SMCC (Pierce Chemical Co., Rockford, IL) at 0.6 mM at room temperature for 1.5 h. The antibody-maleimide is purified on a column of Sephadex G-25 equilibrated in 50 mM potassium phosphate, 150 mM sodium chloride, pH 7.0. The purified antibody-maleimide (2.5 ml) at 5 mg/ml is reacted with an excess of sulfo silicon di[(1,6-diphenyl-2,3-naphthalocyanine] diphthalocyanine bis ((thiopropionamido)butyl dimethylsilyloxide) (2.5 ml) at 0.6 mM at room temperature for 3 h. A solution of N-ethyl maleimide in water is then added to a final concentration of 3 mM and the solution is stirred for an additional 30 min. The antibody-hybrid phthalocyanine derivative is purified on a Sephadex G-25 column equilibrated in 50 mM potassium phosphate, 150 mM sodium chloride, 10 mg/ml bovine serum albumin, pH 7.0.

### Example 39

### Preparation of a Conjugate of Sulfonated Hybrid Phthalocyanine Derivative and a Ligand Analogue

In one embodiment the ligand analogue is morphine. Morphine-HCTL (see U.S. Patent 5,089,391, example 4, incorporated by reference) is hydrolyzed in 0.12 M potassium carbonate/40% (v/v) aqueous methanol at 20 mM at room temperature for 5 min. The solution is then adjusted to pH 7.0 with 1 N hydrochloric acid and diluted to 5 mM with 50 mM potassium phosphate, pH 7.0. A homobifunctional cross linker, (bis-maleimidohexane, Pierce Chemical Co., Rockford, IL) in 50 mM potassium phosphate, pH 7.0, is added to a final concentration of 50 mM. The solution is stirred at room temperature for 1 h and the morphine-maleimide derivative is purified on a reversed phase C₁₈ column using a linear gradient of 50 mM potassium phosphate, pH 7 and methanol. The morphine-maleimide solution in 50 mM potassium phosphate, pH 7.0, is added to a solution of sulfo silicon di[(1,6-diphenyl-2,3-naphthalocyanine] diphthalocyanine bis ((thiopropionamido)butyl dimethylsilyloxide) in 50 mM potassium phosphate, pH 7.0, so that the final concentrations are 10 mM and 2 mM, respectively. The solution is stirred at room temperature for 3 h and the sulfonated hybrid phthalocyanine-morphine derivative is purified on a reversed phase C₁₈ column using a linear gradient of 10 mM potassium phosphate, pH 7.0 and methanol.

It must be noted that as used herein and in the appended claims, the singular forms "a," "and," and "the" include plural referents unless the context clearly dictates otherwise. Thus, for example, reference to "a formulation" includes mixtures of different formulations and reference to "the method of treatment" includes reference to equivalent steps and methods known to t hose skilled in the art, and so forth.

Unless defined otherwise, all technical and scientific terms used herein have the same meaning as commonly understood by one of ordinary skill in the art to which this invention belongs. Although any methods and materials similar to equivalent to those described herein can be used in the practice or testing of the invention, the preferred methods and materials are now described.

## Claims

1. A water soluble hybrid phthalocyanine derivative.

2. A derivative of claim 1 wherein the derivative is selected from the group consisting of:
silicon[di(1,6-diphenyl-2,3-naphthalocyanine)] diphthalocyanine bis[poly(ethylene glycol)methyl ether];
silicon[di(1,6-diphenyl-2,3-naphthalocyanine)] diphthalocyanine bis[poly(ethylene glycol)];
silicon[di(1,6-diphenyl-2,3-naphthalocyanine)] diphthalocyanine [poly(ethylene glycol)][poly(ethylene glycol)acetylthiopropionate] ;
silicon[di(1,6-diphenyl-2,3-naphthalocyanine)]di(2,3-dicarboxyphthalocyanine)dihydroxide;
silicon[di(1,6-diphenyl-2,3-naphthalocyanine)]di(2,3-dicarboxyphthalocyanine) bis[poly(ethylene glycol)methyl ether] ;
sulfo silicon di[(1,6-diphenyl-2,3-naphthalocyanine] diphthalocyanine dihydroxide;
silicon[di(1,6-diphenyl-2,3-naphthalocyanine)] diphthalocyanine [poly(ethylene glycol)] [poly(ethylene glycol) thiopropionate];
sulfo silicon di[(1,6-diphenyl-2,3-naphthalocyanine] diphthalocyanine[-2-butyrothiolactone)amidomethoxide]hydroxide;
sulfo silicon di[(1,6-diphenyl-2,3-naphthalocyanine] diphthalocyanine[N-(cysteine)amidomethoxide]hydroxide;
sulfo[2¹,2⁶,12¹,12⁶-tetraphenyldinaphtho[b,l]-7,17-dibenzo[g,q]-5,10,15,20-tetraazoporphyrinato]silicon dihydroxide;
sulfo[2¹,2⁶,12¹,12⁶-tetraphenyldinaphtho[b,l]-7,17-dibenzo[g,q]-5,10,15,20-tetraazoporphyrinato]silicon bis(4-aminobutyldimethyl silyloxide);
sulfo[2¹,2⁶,12¹,12⁶-tetraphenyldinaphtho[b,l]-7,17-dibenzo[g,q]-5,10,15,20-tetraazoporphyrinato]silicon bis (3-amino-propyl diisopropylsiloxide) ;
sulfo[2¹,2⁶,12¹,12⁶-tetraphenyldinaphtho[b,l]-7,17-dibenzo[g,q]-5,10,15,20-tetraazoporphyrinato]silicon bis-[(10-carbomethoxydecyl)dimethyl silyloxide];
sulfo[2¹,2⁶,12¹,12⁶-tetraphenyldinaphtho[b,l]-7,17-dibenzo[g,q]-5,10,15,20-tetraazoporphyrinato]silicon bis(7-oct-1-enyldimethylsilyloxide);
sulfo[2¹,2⁶,12¹,12⁶-tetraphenyldinaphtho[b,l]-7,17-dibenzo[g,q]-5,10,15,20-tetraazoporphyrinato]silicon bis[N-succinamido) aminobutyldimethyl silyloxide;
sulfo[2¹,2⁶,12¹,12⁶-tetraphenyldinaphtho[b,l]-7,17-dibenzo[g,q]-5,10,15,20-tetraazoporphyrinato]silicon bis [4[(acetylthiopropionamido)butyl]dimethylsilyloxide]; and
sulfo[2¹,2⁶,12¹,12⁶-tetraphenyldinaphtho[b,l]-7,17-dibenzo[g,q]-5,10,15,20-tetraazoporphyrinato]silicon bis [4[(thiopropionamido) butyl]dimethylsilyloxide].

3. A fluorescent conjugate comprising a hybrid phthalocyanine derivate of claim 1 or 2 and a ligand receptor.

4. The fluorescent conjugate of claim 3, wherein the ligand receptor is an antibody.

5. The fluorescent conjugate of claim 4, wherein the antibody specifically binds to human chorionic gonadotropin.

6. A fluorescent conjugate comprising a hybrid phthalocyanine derivative of claim 1 or 2 and a ligand analogue.

7. The fluorescent conjugate of claim 6, wherein the ligand analogue is morphine.

8. A method for determining the presence or amount of at least one target ligand capable of competing with a ligand analogue conjugate for binding sites available on a ligand receptor, said ligand analogue conjugate comprising at least one ligand analogue coupled to a signal development element, said signal development element comprising a water soluble phthalocyanine derivate according to claim 1 or claim 2, in a fluid sample suspected of containing said target ligand comprising the steps of:
a. contacting said fluid sample with said ligand analogue conjugate and said ligand receptor to form a homogeneous reaction mixture;
b. detecting bound or unbound ligand analogue conjugates in said reaction mixture using said water soluble phthalocyanine derivate; and,
c. relating the detectable signal to the presence or amount of said target ligand in said fluid sample.

9. A method of determining the presence or amount of at least one ligand in a fluid sample suspected of containing said target ligand comprising the steps of:
a. contacting said fluid sample with a receptor said receptor coupled to a signal development element comprising a water soluble phthalocyanine derivative according to claim 1 or claim 2, so that said receptor specifically binds said target ligand to form a homogeneous reaction mixture;
b. detecting bound receptor in said reaction mixture using said water soluble phthalocyanine derivative; and,
c. relating the detectable signal to the presence or amount of said target ligand in said fluid sample.

## Patentansprüche

1. Ein wasserlösliches Hybrid-Phthalocyaninderivat.

2. Ein Derivat gemäß Anspruch 1, wobei das Derivat aus der Gruppe ausgewählt ist, bestehend aus:
Silizium[di(1,6-diphenyl-2,3-naphthalocyanin)] diphthalocyanin bis[poly(ethylenglycol)methylether];
Silizium[di(1,6-diphenyl-2,3-naphthalocyanin)] diphthalocyanin bis[poly(ethylenglycol)];
Silizium[di(1,6-diphenyl-2,3-naphthalocyanin)] diphthalocyanin [poly(ethylenglycol)][poly(ethylenglycol)-acetylthiopropionat];
Silizium[di(1,6-diphenyl-2,3-naphthalocyanin)]di(2,3-dicarboxyphthalocyanin)dihydroxid;
Silizium[di(1,6-diphenyl-2,3-naphthalocyanin)]di(2,3-dicarboxyphthalocyanin) bis[poly(ethylenglycol)methylether] ;
Sulfosilizium di[(1,6-diphenyl-2,3-naphthalocyanin] diphthalocyanindihydroxid;
Silizium[di(1,6-diphenyl-2,3-naphthalocyanin)] diphtahlocyanin [poly(ethylenglycol)][poly(ethylenglycol) thiopropionat;
Sulfosilizium di[(1,6-diphenyl-2,3-naphthalocyanin] diphthalocyanin[-2-butyrothiolacton)amidomethoxid]hydroxid;
Sulfosilizium di[(1,6-diphenyl-2,3-naphthalocyanin] diphthalocyanin[N-(cystein)amidomethoxid]hydroxid;
Sulfo[2¹,2⁶,12¹,12⁶-tetraphenyldinaphtho[b,l]-7,17-dibenzo[g,q]-5,10,15,20-tetraazoporphyrinato]-siliziumdihydroxid;
Sulfo[2¹,2⁶,12¹,12⁶-tetraphenyldinaphtho[b,l]-7,17-dibenzo[g,q]-5,10,15,20-tetraazoporphyrinato]silizium bis(4-aminobutyldimethylsilyloxid) ;
Sulfo[2¹,2⁶,12¹,12⁶-tetraphenyldinaphtho[b,l]-7,17-dibenzo[g,q]-5,10,15,20-tetraazoporphyrinato]silizium bis(3-amino-propyldiisopropylsilyloxid] ;
Sulfo[2¹,2⁶,12¹,12⁶-tetraphenyldinaphtho[b,l]-7,17-dibenzo[g,q]-5,10,15,20-tetraazoporphyrinato]silizium bis-[(10-carbomethoxydecyl)dimethylsilyloxid;
Sulfo [2¹,2⁶,12¹,12⁶-tetraphenyldinaphtho[b,l]-7,17-dibenzo[g,q]-5,10,15,20-tetraazoporphyrinato]silizium bis[7-oct-1-enyldimethylsilyloxid) ;
Sulfo[2¹,2⁶,12¹,12⁶-tetraphenyldinaphtho[b,l]-7,17-dibenzo[g,q]-5,10,15,20-tetraazoporphyrinato]silizium bis(N-succinamido)aminobutyldimethylsilyloxid;
Sulfo[2¹,2⁶,12¹,12⁶-tetraphenyldinaphtho[b,l]-7,17-dibenzo[g,q]-5,10,15,20-tetraazoporphyrinato]silizium bis[4[(acetylthiopropionamido)butyl]dimethylsilyloxid]; und
Sulfo [2¹,2⁶,12¹,12⁶-tetraphenyldinaphtho[b,l]-7,17-dibenzo[g,q]-5,10,15,20-tetraazoporphyrinato]silizium bis[4[(thiopropionamido)butyl]dimethylsilyloxkid].

3. Ein fluoreszierendes Konjugat, aufweisend ein Hybrid-Phthalocyaninderivat gemäß Anspruch 1 oder 2 und einen Ligandenrezeptor.

4. Das fluoreszierende Konjugat gemäß Anspruch 3, wobei der Ligandenrezeptor ein Antikörper ist.

5. Das fluoreszierende Konjugat gemäß Anspruch 4, wobei der Antikörper das menschliche chorionische Gonadotropin spezifisch bindet.

6. Ein fluoreszierendes Konjugat, aufweisend ein Hybrid-Phthalocyaninderivat gemäß Anspruch 1 oder 2 und ein Ligandenanalog.

7. Das fluoreszierende Konjugat gemäß Anspruch 6, wobei das Ligandenanalog Morphin ist.

8. Ein Verfahren zum Feststellen der Anwesenheit oder der Menge von mindestens einem Zielligand, der imstande ist, mit einem Ligandenanalog-Konjugat für Bindungsstellen, die auf einem Ligandenrezeptor verfügbar sind, zu konkurrieren, wobei das Ligandenanalog-Konjugat mindestens ein Ligandenanalog, das an einem Signalentwicklungselement gekoppelt ist, aufweist, wobei das Signalentwicklungselement ein wasserlösliches Phthalocyaninderivat gemäß Anspruch 1 oder Anspruch 2 in einer flüssigen Probe aufweist, bei der man vermutet, dass sie den Zielligand enthält, aufweisend folgende Schritte:
a. Kontaktieren der flüssigen Probe mit dem Ligandenanalogkonjugat und dem Ligandenrezeptor, um eine homogene Reaktionsmischung zu bilden;
b. Detektieren gebundener oder nicht gebundener Ligandenanalogkonjugate in der Reaktionsmischung unter Verwendung des wasserlöslichen Phthalocyaninderivats; und
c. Inbeziehungbringen des detektierbaren Signals mit der Anwesenheit oder der Menge des Zielliganden in der flüssigen Probe.

9. Ein Verfahren zum Feststellen der Anwesenheit oder der Menge mindestens eines Ligands in einer flüssigen Probe, bei der man vermutet, dass sie den Zielligand enthält, aufweisend folgende Schritte:
a. Kontaktieren der flüssigen Probe mit einem Rezeptor, wobei der Rezeptor an einem Signalentwicklungselement gekoppelt ist, der ein wasserlösliches Phthalocyaninderivat gemäß Anspruch 1 oder Anspruch 2 aufweist, sodass der Rezeptor den Zielligand spezifisch bindet, um eine homogene Reaktionsmischung zu bilden;
b. Detektieren eines gebundenen Rezeptors in der Reaktionsmischung unter Verwendung des wasserlöslichen Phthalocyaninderivates; und
c. Inbeziehungbringen des detektierbaren Signals mit der Anwesenheit oder der Menge des Zielligands in der flüssigen Probe.

## Revendications

1. Un dérivé de phthalocyanine hybride hydrosoluble.

2. Un dérivé selon la revendication 1, dans lequel le dérivé est choisi dans le groupe constitué par :
silicium [di(1,6-diphényl-2,3-naphthalocyanine)]diphthalocyanine bis [poly(éthylène glycol)méthyl éther] ;
silicium [di(1,6-diphényl-2,3-naphthalocyanine)]diphthalocyanine bis [poly(éthylène glycol)] ;
silicium [di(1,6-diphényl-2,3-naphthalocyanine)]diphthalocyanine [poly(éthylène glycol)] [poly(éthylèneglycol)acéthylthiopropionate] ;
silicium [di(1,6-diphényl-2,3-naphthalocyanine)]di-2,3-dicarboxyphthalo-cyanine)-dihydroxyde ;
silicium [di(1,6-diphényl-2,3-naphthalocyanine)]di (2,3-dicarboxyphthalo-cyanine bis [poly(éthylène glycol)méthyléther] ;
sulfo silicium [di(1,6-diphényl-2,3-naphthalocyanine]diphthalocyanine dihydroxyde ;
silicium [di(1,6-diphényl-2,3-naphthalocyanine)]diphthalocyanine [poly(éthylène glycol)] [poly(éthylène glycol)thiopropionate] ;
sulfo silicium [di(1,6-diphényl-2,3-naphthalocyanine)]diphthalocyanine [2-butyrothiolactone)amidométhoxyde]hydroxyde ;
sulfo silicium [di(1,6-diphényl-2,3-naphthalocyanine)]diphthalocyanine [N-(cystéine)amidométhoxyde]hydroxyde ;
sulfo [2¹,2⁶,12¹,12⁶-tétraphényldinaphtho [b,l]-7,17-dibenzo [g,q]-5,10,15,20-tétraazoporphyrinato]silicium dihydroxyde ;
sulfo [2¹,2⁶,12¹,12⁶-tétraphényldinaphtho [b,l]-7,17-dibenzo [g,q]-5,10,15,20-tétraazoporphyrinato]silicium bis (4-aminobutyldiméthyl silyloxyde) ;
sulfo [2¹,2⁶,12¹,12⁶-tétraphényldinaphtho [b,l]-7,17-dibenzo [g,q]-5,10,15,20-tétraazoporphyrinato]silicium bis (3-aminopropyl diisopropylsiloxyde) ;
sulfo [2¹,2⁶,12¹,12⁶-tétraphényldinaphtho [b,l]-7,17-dibenzo [g,q]-5,10,15,20-tétraazoporphyrinato]silicium bis-[(10-carbométhoxydécyl)diméthyl silyloxyde] ;
sulfo [2¹,2⁶,12¹,12⁶-tétraphényldinaphtho [b,l]-7,17-dibenzo [g,q]-5,10,15,20-tétraazoporphyrinato]silicium bis (7-oct-1-ényldiméthylsilyloxyde) ;
sulfo [2¹,2⁶,12¹,12⁶-tétraphényldinaphtho [b,l]-7,17-dibenzo [g,q]-5,10,15,20-tétraazoporphyrinato]silicium bis [N-succinamido) aminobutyldiméthyl silyloxyde ;
sulfo [2¹,2⁶,12¹,12⁶-tétraphényldinaphtho [b,l]-7,17-dibenzo [g,q]-5,10,15,20-tétraazoporphyrinato]silicium bis [4[(acétylthiopropionamido)-butyl]-diméthylsilyloxyde] ; et
sulfo [2¹,2⁶,12¹,12⁶-tétraphényldinaphtho [b,l]-7,17-dibenzo [g,q]-5,10,15,20-tétraazoporphyrinato]silicium bis [4 [(thiopropionamido)-butyl]-diméthylsilyloxyde].

3. Un conjugué fluorescent comprenant un dérivé de phthalocyanine hybride selon l'une quelconque des revendications 1 ou 2 et un récepteur ligand.

4. Le conjugué fluorescent selon la revendication 3, dans lequel le récepteur ligand est un anticorps.

5. Le conjugué fluorescent selon la revendication 4, dans lequel l'anticorps se fixe spécifiquement sur la gonadotropine chorionique humaine.

6. Un conjugué fluorescent comprenant un dérivé de phthalocyanine hybride selon l'une quelconque des revendications 1 ou 2 et un analogue de ligand.

7. Le conjugué fluorescent selon la revendication 6, dans lequel l'analogue de ligand est la morphine.

8. Une méthode de détermination de la présence ou de la quantité d'au moins un ligand cible capable d'entrer en compétition avec un conjugué d'analogue de ligand pour les sites de fixation disponibles sur un récepteur de ligand, ledit conjugué d'analogue de ligand comprenant au moins un analogue de ligand associé à un élément de développement de signal, ledit élément de développement de signal comprenant un dérivé de phthalocyanine hydrosoluble selon l'une quelconque des revendications 1 ou 2, dans un échantillon de fluide suspecté de renfermer ledit ligand cible comprenant les étapes de :
a. mise en contact dudit échantillon de fluide avec ledit conjugué d'analogue de ligand et ledit récepteur de ligand pour former un mélange réactionnel homogène ;
b. détection des conjugués analogues de ligand liés ou non liés dans ledit mélange réactionnel en utilisant ledit dérivé de phthalocyanine hydrosoluble ; et
c. association du signal détectable à la présence ou à la quantité dudit ligand cible dans ledit échantillon de fluide.

9. Une méthode de détermination de la présence ou de la quantité d'au moins un ligand dans un échantillon de fluide suspecté de renfermer ledit ligand cible comprenant les étapes de :
a. mise en contact dudit échantillon de fluide avec un récepteur, ledit récepteur étant couplé à un élément de développement de signal comprenant un dérivé de phthalocyanine hydrosoluble selon la revendication 1 ou 2, de façon que ledit récepteur fixe spécifiquement ledit ligand cible pour former un mélange réactionnel homogène ;
b. détection du récepteur lié dans ledit mélange réactionnel en utilisant ledit dérivé de phthalocyanine hydrosoluble ; et
c. association du signal détectable à la présence ou à la quantité dudit ligand cible dans ledit échantillon de fluide.
